(19) Europäisches Patentamt
European Patent Office
Office européen des brevets

(11) EP 1 423 541 B1

(12) EUROPEAN PATENT SPECIFICATION

(45) Date of publication and mention of the grant of the patent:
**19.07.2006 Bulletin 2006/29**

(21) Application number: **02797620.8**

(22) Date of filing: **24.08.2002**

(51) Int Cl.:
*C12Q 1/68* (2006.01)

(86) International application number:
**PCT/EP2002/009496**

(87) International publication number:
**WO 2003/020967 (13.03.2003 Gazette 2003/11)**

# (54) A METHOD FOR THE DETERMINATION OF MULTIPLE ANALYTES

VERFAHREN ZUR BESTIMMUNG VON MEHRERE ANALYTEN

PROCEDE POUR DETERMINER DES ANALYTES MULTIPLES

(84) Designated Contracting States:
**AT BE BG CH CY CZ DE DK EE ES FI FR GB GR IE IT LI LU MC NL PT SE SK TR**

(30) Priority: **28.08.2001 EP 01120466**

(43) Date of publication of application:
**02.06.2004 Bulletin 2004/23**

(73) Proprietors:
- **Roche Diagnostics GmbH**
  **68305 Mannheim (DE)**
  Designated Contracting States:
  **DE**
- **F.HOFFMANN-LA ROCHE AG**
  **4070 Basel (CH)**
  Designated Contracting States:
  **AT BE BG CH CY CZ DK EE ES FI FR GB GR IE IT LI LU MC NL PT SE SK TR**

(72) Inventors:
- **WEINDEL, Kurt**
  **82407 Wielenbach-Hardt (DE)**
- **KRAISS, Stefan**
  **Pleasanton, CA 94566 (US)**
- **BERGMANN, Frank**
  **82393 Iffeldorf (DE)**
- **JOSEL, Hans-Peter**
  **82362 Weilheim (DE)**
- **HEINDL, Dieter**
  **82327 Tutzing (DE)**

(56) References cited:
**US-A- 5 759 781**

- **SPEICHER M R ET AL: "KARYOTYPING HUMAN CHROMOSOMES BY COMBINATORIAL MULTI-FLUOR FISH" NATURE GENETICS, NEW YORK, NY, US, vol. 12, April 1996 (1996-04), pages 368-375, XP000930084 ISSN: 1061-4036**
- **VET J A M ET AL: "Multiplex detection of four pathogenic retroviruses using molecular beacons" PROCEEDINGS OF THE NATIONAL ACADEMY OF SCIENCES OF USA, NATIONAL ACADEMY OF SCIENCE. WASHINGTON, US, vol. 96, 1999, pages 6394-6399, XP002145609 ISSN: 0027-8424**
- **MERCIER BERNARD ET AL: "Simultaneous screening for HBV DNA and HCV RNA genomes in blood donations using a novel TaqMan PCR assay." JOURNAL OF VIROLOGICAL METHODS, vol. 77, no. 1, January 1999 (1999-01), pages 1-9, XP002193782 ISSN: 0166-0934**
- **TONG ANTHONY K ET AL: "Combinatorial fluorescence energy transfer tags for multiplex biological assays." NATURE BIOTECHNOLOGY, vol. 19, no. 8, August 2001 (2001-08), pages 756-759, XP002193783 August, 2001 ISSN: 1087-0156**
- **MENG Q ET AL: "Automated multiplex assay system for simultaneous detection of hepatitis B virus DNA, hepatitis C virus RNA, and human immunodeficiency virus type 1 RNA." JOURNAL OF CLINICAL MICROBIOLOGY, vol. 39, no. 8, August 2001 (2001-08), pages 2937-2945, XP002193784 ISSN: 0095-1137**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- SAMIOTAKI M ET AL: "SEVEN-COLOR TIME-RESOLVED FLUORESCENCE HYBRIDIZATION ANALYSIS OF HUMAN PAPILLOMA VIRUS TYPES" ANALYTICAL BIOCHEMISTRY, ACADEMIC PRESS, SAN DIEGO, CA, US, vol. 253, no. 2, 15 November 1997 (1997-11-15), pages 156-161, XP000721248 ISSN: 0003-2697 cited in the application
- JOSEFSSON AGNETHA ET AL: "Detection and quantitation of human papillomavirus by using the fluorescent 5' exonuclease assay." JOURNAL OF CLINICAL MICROBIOLOGY, vol. 37, no. 3, March 1999 (1999-03), pages 490-496, XP002193785 ISSN: 0095-1137
- RIED T ET AL: "SIMULTANEOUS VISUALIZATION OF SEVEN DIFFERENT DNA PROBES BY IN SITU HYBRIDIZATION USING COMBINATORIAL FLUORESCENCE AND DIGITAL IMAGING MICROSCOPY" PROCEEDINGS OF THE NATIONAL ACADEMY OF SCIENCES OF USA, NATIONAL ACADEMY OF SCIENCE. WASHINGTON, US, vol. 89, 1 February 1992 (1992-02-01), pages 1388-1392, XP002073155 ISSN: 0027-8424 cited in the application

## Description

This invention is directed to methods for the determination of multiple analytes using analyte specific binding partners wherein the binding partners are labeled with less different labels than different binding partners used. Additionally, this invention is directed to compositions of matter containing these differently labeled binding partners and the use of such compositions for the determination of multiple analytes as well as suitable kits.

## Background of the invention

The determination of an analyte in a sample has aquired especial importance particularly in the field of health care, nutriation and ecology. Depending on the analyte different methods for determination can be used. Small molecules like metal ions, sugar monomers, amino acids often are determined by their chemical or physical properties. Analytes having a higher molecular weight like proteins and nucleic acid polymers can also be determined using binding partners having a specific affinity to them. Useful pairs of binding partners are antibody - antigen, substrate - enzyme, nucleic acid - complementary nucleic acid, sugar - lectin. In case it is intended to determine a specific protein for which one knows that it exhibits antigenic properties a specific antibody can be used for the determination. For the determination of a specific nucleic acid sequence a nucleic acid probe having a complementary sequence can be used. Such binding assays and suitable protocols are well known in the art and are fully explained in the literature, see for example Sambrook et al., 1985, Molecular Cloning - A Laboratory Manual, Coldspring Harbor Laboratory, Cold Spring Harbor, New York; Nucleic Acid Hybridization (B.D. Hames and S.J. Higgins, eds. 1984) and a series Methods in Enzymology (Academic Press, Inc.).

In order to allow the detection of the specific binding partner - analyte complexes it is very common to label the binding partners, whereby these labels can be detected directly or indirectly using additional reagents which bind to these labels (for example using biotin as a label, which can be detected using a avidin - horseraddich peroxidase conjugate and a suitable enzyme - substrate reaction for detection). Suitable labels are known in the art.

For many cases, especially in the diagnostic field it is necessary to determine two or more analytes in order to get a complete picture about a distinct situation. For example regarding a patient who might have a Chlamydia trachomatis infection, a doctor normally will not only check for a Chlamydia trachomatis but also for a Neisseria gonorrhoeae infection. Also when establishing the HLA pattern of a patient one need to determine the alleles of several different HLA loci.

When conducting a multiple analyte binding assay one has to pay attention to the labels used. In a first case the same label is used for all different binding partners. In this case two alternative formats have to be differentiated. If all binding reactions are conducted together in one reaction a positive result does only show that there is at least one analyte present in the sample, but not which one it is. As an alternative one can separate each binding reaction by conducting sequential reactions or using parallel reaction formats. Suitable formats are for example microtiterplate- or dot-blot-assays. Methods using such formats are known in the art. Such formats are time consuming and require a lot of handling steps, which could result in increased costs and contamination risks. More sophisticated methods using biochips are very expensive and are difficult to handle with regard to contaminations especially in the field of nucleic acid amplification.

In order to allow the discrete detection of several analytes within only one binding and/or detection reaction the different specific binding partners can be labeled using different labels, which can be separately detected. Such labels and useable detection methods are well known in the art. For example one can use labels which can be detected by its optical emission spectrum, whereby each of the label has a different emission spectrum. Although, it has to be noted that such assays are in reality limited to the determination of only a few analytes, because there is not an unlimited number of suitable labels which are detectable separately with high sensitivity, and sufficiently stable. In case of suitable rare earth labels, expensive and highly sophisticated detectors for time-resolved fluorimetry are necessary . These limitations are of special importance with regard to homogeneous detection methods. Such formats normally suffer from a higher signal background due to missing washing steps to eliminate binding partners which are not specifically bound to an analyte, which makes it difficult to interprete the multiple detection signals properly.

Multilabelling in conjunction with detectors featuring a plurality of optical channels has been used by many investigators. For instance, Vet et al. (PNAS 96, 6394-6399 (1999)) use methodology, in which 4 specific probes (Molecular Beacons) complementary to 4 different targets are labelled with 4 different reporter dyes, 1 dye each. The entire fluorescence spectrum of each dye is stored in the computer and used to interpret complex multiplex results. This entails need for quite sophisticated software in order to cope with the demand to be able to resolve co-infections via spectral contribution analysis. This includes normalisation of fluorescent signals from different dyes individually for each particular dye (optical channel) on maximum gain, i.e. normalisation of channel-specific growth curves pertinent to the corresponding plateau, not across different optical channels on a reference dye in order to be able to analyse signal distribution on a common basis. Other examples include Josephsson et al. (J. Clin. Microbiol. 37/3, 490-496 (1999); up to 3 differently labelled Molecular Beacons for up to 3 targets per assay) and Mercier et al. (J. Virol. Methods 77, 1-9 1999); 2 differently labelled probes for 2 targets per assay).

In order to increase the number of analytes detectable with a distinct number of labels also combinations of labels for labeling of binding partners can be used. In addition to binding partners having one distinct label attached also binding partners are used which are coupled with two or more labels using the same kind of labels also coupled to the single - labeled binding partners. Using for example 3 different labels 7 combinations of labels are possible by which also 7 different analytes can be detected. An in-situ-hybridization method using a similar principle is described by T. Ried et al., Proc. Natl. Acad. Sci USA, Vol. 89, pp. 1388 -1392 (1992). A similar method for determination of 7 different Human Papilloma Virus types in a sample using PCR and a subsequent probe hybridization assay are described by Samiotaki et al., Analytical Biochemistry Vol. 253, p. 156 - 161 (1997). For this purpose, oligonucleotides are 5'-terminally modified by a plurality of chelator moieties. The individual chelate units are subsequently filled with either one and the same sort of rare earth ion, or a precisely adjusted mixture of up to 3 rare earth ions. Thus, in case of > 1 sort of ion being fixed by complexation, multiple fluorescent rare earth chelates ("dye mixtures") are coupled to one and the same probe molecule. Such probes are not easy to synthesise, and poorly applicable (if at all) in homogeneous amplification/ detection formats.

There are a number of other technological approaches to combinatorial labelling. Ballard et al. (US 5,759,781), Speicher et al. (Nat. Genetics 12,368-375 (1996)) and Ried et al. (PNAS 89, 1388-1392 (1992)) have developed assay formats, in which each specific probe is known by its labelling signature, as all copies of a given probe carry 1 or more distinct fluorophors in a combinatorial fashion. Labelling is done by means of nick translations, which is less precise in terms of number of labels incorporated per probe molecule than chemical oligonucleotide synthesis, i.e. the methods doesn't lend itself well to accurately analysing discrete distributions. Therefore an expert would not use such probes in nucleic acid amplification methods, especially not in homogeneous amplification methods.

The so-called CFET probes described by Tong et al. (Nat. Biotechnology 19, 756-759 (2001)) have a principally different design and mode of application. 1 to 3 dyes are coupled 5'-terminal per probe molecule, with spacer units in precisely adjusted number interspersed. The oligonucleotide moiety follows after the last dye or spacer towards the 3'-end. Thus, there is a mixture of different probe types as with Ballard et al. The spacers function as tuners for electrophoretic mobility, and for resonance intensity as well. The latter affects the fluorescent signature of the particular probe. Probes are excited at a common wavelength, and the recorded signal is resolved by the position in the capillary electropherogram *and* by overlay of the complete emission spectra at the particular position and calculation of relative contributions from components of the label set directly as digital ratios. Again, there is no signal distribution analysis of corrected & normalised signals *per se,* nor dynamically along the reaction coordinate, no "same sequence / different label" oligonucleotide design, and no signal generation as a consequence of a biochemical reaction altering probe configuration or ~ constitution.

The labeling of binding partners with more than one label per binding partner molecule bears some disadvantages. Such multiple labeled binding partners are rather demanding (and costly) in synthesis and steric hindering of the labels can occur which might decrease the efficiency of the binding of the binding partner to ist analyte or the detection of the attached labels. Also interactions between the labels attached to the binding partner might occur, which could lead to wrong results. This could be relevant for example when using optical labels. It is certainly of especial importance when conducting homogeneous detection methods using fluorescence energy transfer labels as for example used in TaqMan assays (US 5210015, EP 0543942).

Therefore, it is an object of the present invention to improve the methods for determination of multiple analytes avoiding all or a part of the disadvantages of the known methods.

US 5,759,781 discloses a method for the determination of more than 3 analytes, human chromosome specific sequences, comprising providing a mixture containing said analytes and more than 3 nucleic acid probes under conditions allowing the specific hybridization and determining the presence of the analytes using these signal intensities. In this mixture, a first probe specific for chromosome 1 is coupled to a first label; a second probe specific for chromosome 2 is coupled to a second label. A first amount of a third probe specific for chromosome Y is coupled to the same label as the first probe, whereas a second amount of the third probe is coupled to the same label as the second probe.

## Summary of the invention

The main aspect of the present invention is related to a method for determination of multiple analytes using less labels than analytes determined. This is achieved by labelling the whole ensemble of specific probe molecules for some targets with a particular dye each, while for other targets one part of the ensemble of specific probes is labelled with a given dye, while another part of the ensemble is labelled with a different dye (same sequence / different label oligonucleotides in admixture). Preferably by performing homogeneous (solution-phase) real-time amplification assays (for example by PCR or TMA), correcting the resulting signal for all kind of relevant noise, and normalising the signal from different labels on a reference dye, targets can be deduced from the distribution of processed signal collected in a multi-channel detector. For this purpose combinations of labels are used.

For example when determining 3 analytes only two different detectable labels are necessary. A first label is attached to a first binding partner specific for the first analyte. A second label is attached to a second binding partner specific for the second analyte. For labeling of the third binding partner specific for the third analyte the same kind of labels as has been coupled to the first and second binding partners are used. One amount of the third binding partner is coupled with the first label. In case of three analytes preferably one half of the third binding partner is labeled this way. Another part of the third binding partner is coupled to the second label, which would be in case of three analytes preferably the remaining half of the third binding partner. Therefore each binding partner specific for this analyte contains a first label or a second label. This results in binding partner molecules each labeled with only one label, preferably enabling multi-colour analysis for resolving multiplex results based on signal generation which is derived from a biochemical reaction and thus provides means for kinetic response recording, while maintaining simple probe designs and state-of-the art instrumentation for detection. In contrast thereto probes are described in the literature that have bound multiple labels to one probe molecule (Samiotaki, M. et al., Analytical Biochemistry 253, 156 -161 (1997)). Such probes are more difficult to synthesize compared with the binding partners according to the present invention.

Different detection efficiencies which may occur for the different labels attached to the third binding partner, can be compensated for in 2 ways. In case a first label has a higher signal output compared to a second label, one can adjust this difference either chemically by mixing the third binding partner coupled to the first label with the third binding partner coupled to the second label in a defined non-1:1 ratio, or mathematically via normalization of signal output to a given label selected as standard. Such different detection efficiencies may be due to the intrinsic properties of the labels, such as absorptivity or quantum yield, but can also result from different coupling efficiencies, or susceptibility to solvent effects. In the latter case the mixing of the different amounts of the third binding partner following the coupling reaction provides a good possibility to adapt such discrepancies.

In addition possible interference which may occur when two or more detectable label are attached to one binding partner molecule is avoided. Therefore, binding partners according to the present invention are compatible with broad spectrum of labels, which also encompass fluorophores and combinations of fluorophores, like classical Förster type labels (Styer and Haugland, Proc. Natl. Acad. Sci. USA 98, 719 (1967)) which can be used for example in homogeneous detection assays like the TaqMan-method for determination of low concentrated nucleic acid analytes.

It should be noted that also an increased number of analytes may be determined using three or even more labels which can be combined in the same way as shown for the two labels. When using three labels up to seven analytes may be determined. Using four different labels even 15 analytes can be detected if applied singular as well as in dual, triplicate and quadruplicate combinations. If applied singular or in dual combinations only (which might be advantageous from a practical point of view), up to 10 different analytes can be differentiates with a set of 4 labels.

Therefore the present invention is related to a method for the determination of at least 3 nucleic acid analytes comprising the steps:

a) Providing a mixture of a sample containing said at least 3 nucleic acid analytes or suspected of containing one or more of said nucleic acid analytes

b) providing at least 3 different nucleic acid sequence specific probes

c) amplifying said nucleic acid analytes

d) choosing reaction conditions which promote the specific binding of said nucleic acid sequence specific probes to said amplified nucleic acid analytes, wherein

- a first of said at least 3 nucleic acid sequence specific probes is specific for a first of said at least 3 nucleic acid analytes and is coupled to a first label,

- a second of said at least 3 nucleic acid sequence specific probes is specific for a second of said at least 3 nucleic acid analytes and is coupled to a second label, which label is separately detectable from the label coupled to said first nucleic acid sequence specific probe, and

- a third of said at least 3 nucleic acid sequence specific probes specific for a third of said at least 3 nucleic acid analytes whereby a first amount of said third nucleic acid sequence specific probe is coupled to the same label as coupled to said first nucleic acid sequence specific probe and a second amount of said third nucleic acid sequence specific probe is coupled to the same label as the second nucleic acid sequence specific probe and said first amount of said third nucleic acid sequence specific probe is not coupled to the same label as coupled to the second nucleic acid sequence specific probe and said second amount of said third nucleic acid sequence specific probe is not coupled to the same label as coupled to the first nucleic acid sequence specific probe,

e) Detecting the signal intensities indicative for said first and second label,

f) Determining the nucleic acid analytes present in said sample using said signal intensities detected in step e).

The present invention is also related to akit for the determination of at least 3 nucleic acid analytes containing in one or more containers

- a first nucleic acid sequence specific probe specific for a first nucleic acid analyte coupled to a first label,
- a second nucleic acid sequence specific probe specific for a second nucleic acid analyte coupled to a second label, which label is separately detectable from the label coupled to said first nucleic acid sequence specific probe,
- a third nucleic acid sequence specific probe specific for a third nucleic acid analyte, whereby a first amount of said third nucleic acid sequence specific probe is coupled to the same label as coupled to said first nucleic acid sequence specific probe and a second amount of said third nucleic acid sequence specific probe coupled to the same label as said second nucleic acid sequence specific probe, and
- reagents for nucleic acid amplification.

Preferred analytes are nucleic acid analytes which can be determined using sequence specific probes. Such nucleic acid analytes can also be amplified nucleic acids using one of several nucleic acid amplification method known in the art, like LCR (U.S. Patent Nos. 5,185,243, 5,679,524 and 5,573,907; EP 0 320 308 Bl; WO 90/01069; WO 89/12696; and WO 89/09835), cycling probe technology (U.S. Patent Nos. 5,011,769, 5,403,711, 5,660,988, and 4,876,187, and PCT published applications WO 95/05480, WO 95/1416, and WO 95/00667), Invader TM technology (U.S. Patent Nos. 5,846,717; 5,614, 402; 5,719,028; 5,541,311; and 5,843,669), Q-Beta replicase technology (U.S. Patent No. 4,786,600) , NASBA (U.S. Patent No. 5,409,818; EP-0 329 822), TMA (U.S. Patent Nos. 5,399,491, 5,888,779, 5,705,365, 5,710,029), SDA (U.S. Patent Nos. 5, 455,166 and 5,130,238) and PCR (US-A-4,683,202), whereas the PCR method is most preferred.

**Brief description of the drawings**

Fig. 1 shows a possible data processing scheme for an automated multiple analyte determination assay using the determination methods described. See also example 2.

**Detailed description of the invention**

Analytes according to the present invention are analytes which can be determined by binding assays known in the art. These are preferably components of samples for medical dignostics or other biological analytics, i.e. in particularly ingredients of body components such as antigens, antibodies, cells or nucleic acids. Such assays can be used for example for determination of infectious agents like bacteria e.g. chlamydia, neisseria and mycobacteria and viruses like HBV, HCV and HIV. Depending on the aim of an assay one can determine the amount of an analyte present in a sample as well as the configuration of an analyte, for example a nucleic acid analyte can be analysed for its allelic form or whether a patient carries a mutated form.

A sample according to the present invention contains the analyte to be determined. With regard to medical diagnostics samples derived from human or animal are preferred, e.g. whole blood, tissue sections, urine, sputum, serum, plasma, buffy coat and smears can be used. Depending on the analyte and the sample it might be necessary to pre-process the sample in order to allow determination of the analyte, for example for most samples nucleic acids need to be extracted in a first step. Such pre-processed samples are also samples according to the present invention.

According to the present invention at least three analytes are determined. This could be for example a pattern of infectious agents in a patient, like the viruses HIV, HBV and HCV, which have to be tested for each blood sample in blood banks. Another example could be the determination of the histocompatibility locus antigene pattern, which consists of several loci and distinct allelic forms.

The analytes are bound by specific binding partners. Depending on the analyte one has to choose specific binding partners. Analytes having antigenic properties can be bound by using specific antibodies. Antibodies in a sample can be determined by using the specific antigens as binding partners. If a nucleic acid analyte should be determined one can use a nucleic acid sequence being complementary to the analyte as specific probe. Further specific binding pairs like substrate - enzyme or sugar - lectin are known in the art, which might also be useful in the described methods.

A nucleic acid analyte is usually brought into available form by processing the original sample with one of various methods. This comprises for example change of pH (alkaline), heating, cyclic changes of temperature (freezing/thawing), change of the physiological growing conditions, use of detergents, chaotropic salts or enzymes (for example proteases or lipases), alone or in combination. For example one can use magnetic glass particles which are able to bind nucleic acid under specific conditions. Suitable particles and protocols are described in WO 96/41811 and WO 01/37291.

It is important to choose reaction conditions which promote the specific binding of the binding partners to the analytes which allows that the specific binding complexes occur but minimize the binding of the binding partners to unrelated reaction and sample components leading to an increased background signal. Such reaction conditions and suitable protocols are known in the art.

A specific nucleic acid binding partner, also called probe, for determination of a nucleic acid analyte or an amplified nucleic acid analyte preferably is an oligonucleotide, but also analogues having for example a peptide-backbone instead of the natural phosphate-sugar backbone (PNA, WO 92/20702) can be used. In order to allow a specific binding of the probe to the analyte the probes are preferably longer than 10 nucleotides, even more preferred have a length of 10 to 40 nucleotides. It is further required that the probe is sufficiently complementary to the analyte sequence. Therefore probes are preferably at least 80 % complementary, more preferred are at least 90 % complementary. In the most preferred case the probes are fully complementary to the analyte. The exact determination of complementarity and homology can be determined by using computer programs such as FastA (Pearson and Lipman, Proc. Natl. Acad. Sci. USA Vol. 85, pp. 2444-2448 (88)).

Depending on the initial concentration of a nucleic acid analyte it might be necessary to amplify the analyte in order to allow its determination. For this purpose several amplification methods are known in the art (as referenced above). Especially when using primer based amplification methods a specific amplification of a nucleic acid analyte is possible. In combination with a probe binding assay an increased specificity of an assay can achieved. Also methods are known in the art, like LCR, which use labeled primers in order to allow the detection of an analyte. Also such methods can be modified according to the present invention.

A primer according to the present invention is a molecule capable of being extended or modified preferably by enzymes, more preferably by a polymerase of for instance procaryotic origin, when hybridized to a nucleic acid template. When using PCR methodology thermostable polymerases like *T. aquaticus* or *T. thermophilus* DNA-polymerase are preferred. These extend primers by adding mononucleotide units from monodesoxyribonucleosidetriphosphates to the 3'-OH-terminal end of said primers. The overall length and base sequence of a primer is dictated by the required specificity of the amplification reaction. Preferred primer lengths for performing PCR are from 10 to 40, most preferred from 15 to 30 base containing subunits, selected from mononucleotides and/or nucleic acid analog monomers. In general primers of that length are also useful for other amplification methods. If more than one primer is used for amplification, for example when using PCR or amplifying multiple target nucleic acids in one reaction, preferably primers are used which cannot hybridize to each other, because they do not contain any stretch of more than 5 consecutive complementary bases.

Labels are generally known to those skilled in the art as being a group which is detectable or can be made detectable for determination the presence of an analyte. Well-known labels are fluorescent labels, like fluoresceine and lanthanide chelates, electrochemiluminescent labels, like ruthenium complexes, or moieties that can be recognized by another molecular entity, like haptens which can be recognized by an antibody raised against this hapten or moieties that can be immobilized, like biotin which can be bound for example to streptavidin coated solid phases, like beads or tubes. Most preferred labels especially with regard to homogenous formats are chromophores. Such chromophores can be used alone or in combination with another chromophore or for example with a non-fluorescent quencher.

In case homogeneous detection formats are used, especially for determination of nucleic acid analytes several formats are known in the art. Method like the TaqMan assay (US Nos. 5,210,015 and 5,487,972) and the kissing probe-assay are based on fluorescent energy transfer of fluorescent dyes (FET, Styer and Haughland Proc. Natl. Acad. Sci. USA Vol. 98, pp. 719- (67)). When brought in close proximity with each other a first fluorophor can interact with a second fluophor or a non-fluorescent quencher. For example, the electromagnetic emission or vibrational excitation of a first fluorescent dye can induce resonance in a second fluorescent dye. Depending on the format used one can for example detect a decreasing light emission of the first fluorescent dye or an increasing light emission of the second fluorescent dye as a mean for the presence of an analyte. Combinations of fluorescent dyes, which can be used for this purpose, are known in the art. Examples are classical dyes suitable for Förster-type Resonance Energy Transfer like Pentamethin-indodicarbocyanin (Cy5) combined with 6-Carboxyfluorescein (6-FAM).

Label in this context means a signal generating entity, which is actually detected in the method of the present invention. With regard to the homogeneous formats described, pairs of fluorochromes are used, which get into resonance with each other and which are detected in these methods as a single detection signal. Although two distinct molecules, for example two fluorochromes, are involved, they function as one single label. In addition, the term label has to be understood that it could also mean that more than one label molecule of a distinct label is coupled to a binding partner molecule.

Depending on the labels used different detectors have to be used for measuring of the signals of the labels. Beside others, especially fluorimeters are widely used for this purpose. Fluorescent labelling is most advantageous in conjunction with homogeneous PCR, as no chemical trigger reagent has to be added for signal generation, in contrast to enzymatic or chemiluminescent labelling techniques. This allows for a closed tube procedure, the most effective way to avoid cross-contamination with amplified material. When determining multiple label signals it is important that the signals are distinguishable from each other. In order to avoid such cross-talk it is preferred when using optical labels that each label has a different emission and/ or extinction-spectrum. Although when using most of the commercially available labels in multiple analyte detection assay at least some cross-talk cannot be avoided. When determining only a few analytes this cross-talk can be compensated by processing the measured signals with computer programs or using more expensive spectral fluorimeters instead of filter based fluorimeters. With regard to homogenous formats especially when using fluorescence energy transfer-based methods the need of additional chromophores even increases the possibilities of interferences. Therefore in reality the number of suitable labels which can be used together in a multiple assay is limited.

According to the present invention at least one of the binding partners used is coupled with a combination of detectable labels, whereby each of these binding partner molecules is coupled to only one detectable label. In contrast probes as described by Samiotaki et al (as referenced above) are labeled with multiple labels (e.g. 10-20 coupled 5'-terminal per oligonucleotide). Due to the proximity of the detectable labels coupled to the same probe molecule the risk of interference between the labels is very high and not all types of labels especially fluorescent dyes can be used.

The methods according to the present invention can be used for determination of an increased number of analytes in a multiple analyte assay and are compatible with a broad range of labels.

By decreasing the number of labels necessary in a multiplex assay the need for exotic labels is avoided and also cheaper detectors like filter based fluorimeters can be used instead of spectral fluorimeters which are much more expensive. This further allows for homogeneous formats, which are of outstanding importance with regard to commercial diagnostic assays due to the limited contamination risks and fewer handling steps.

As indicated above, three labels combined in different combinations can be coupled with up to seven different specific binding partners allowing the distinct determination of six different analytes. This is visualized in the table below. P stands for Parameter = analyte, a channel corresponds to an optical tract as part of a detector optimised to capture the signal of a particular detectable label. The table shows the expected percentage distribution of signal over all three channels, being normalized either chemically or mathematically. Considered is the case that of the analytes P1 to P7 only one at a time would be present in a sample. The labeling of the different binding partners are: type P1 - Label11 (signal in channel 1, P2 - Label 2 (signal in channel 2), P3 - label 3(signal in channel 3), P4 - half amount label 1, half amount label 2, P5 - half amount label 1, half amount label 3, P6 - half amount label 2, half amount label 3 and P7-1/3 amount label 1, 1/3 amount label 2, 1/3 amount label 3.

| Parameter | Channel | | |
|---|---|---|---|
| | 1 | 2 | 3 |
| P1 | 100 | | |
| P2 | | 100 | |
| P3 | | | 100 |
| P4 | 50 | 50 | |
| P5 | 50 | | 50 |
| P6 | | 50 | 50 |
| P7 | 33 | 33 | 33 |

If a signal can be only measured in one channel a dear indication for the presence of one of the analytes of parameter 1, 2 or 3 is given (see table). If signals occur in two channels in a 50: 50 ratio there is a strong indication for an analyte of parameter 4, 5 or 6 as indicated in the table. A signal ratio of 33:33:33 would indicate that a P7 analyte is present in the sample. However this reflects an idealized situation. Real signals may deviate and often need to be normalized in order to compensate for example different detection efficiencies of the labels, background- and cross talk-signals.

The different labels coupled to the binding partners should not change the binding efficiency of a binding partner, which might also be adjustable by varying the ratios of binding partner bound to the first label: binding partner bound to the second label. Also different detection efficiencies can be adjusted by this way.

A 50: 50 signal ratio might also occur in case of the presence of two analytes. However this would probably be extremely rare, since their occurrence would depend on either a 1:1 co-infection in terms of number of starting molecules and identical extraction / amplification / detection efficiencies yielding a 50:50 signal distribution into 2 channels, or on different numbers of starting molecules in conjunction with extraction / amplification / detection efficiencies that precisely counter-balance the differences in titer and again yield a 50:50 distribution. Thus, co-infections of 2 parameters indicated by single labeled probe, respectively, e.g. P1 and P2, should result in an uneven distribution of>>50:<<50: 0 or <<50: >>50: 0 and so be distinguishable from e.g. P4 indicated by an even distribution of 50: 50: 0. However such results could also derived from samples co-infected with two agents for example P4 and P1.

In order to further decrease the risk of ambiguous results and possible errors in interpretation of the measured signals it is possible to arrange the parameters in a way that signals occurring in two or more channel can be clearly assigned to one possible analyte distribution in the sample as explained in example 1. In addition one can run an additional assay especially testing for the remaining possible analyte distributions which may be present in the sample. Such a sequential method is very economical as it allows to determine a distinct analyte distribution in most samples in a first run, whereas ambiguous samples can be re-evaluated in a second run using for example a multiple analyte assay with a reduced number of analytes (for which an unambiguous one label - one binding partner combination should be possible). Furthermore regarding nucleic acid analytes and amplified nucleic acid analytes one could for example determine the melting curve of the analyte-probe hybridization complexes or of the amplified nucleic acid, which allows distinguishing the different analytes and coming to an unambiguous result.

Also kits for determination of at least three analytes are subject of the present invention. Such kits containing in one or more containers

- a first binding partner specific for a first analyte coupled to a first label,

- a second binding partner specific for a second analyte coupled to a second label, which label is separately detectable from the label coupled to said first binding partner, and

- a third binding partner specific for a third analyte, whereby a first amount of said third binding partner is coupled to the same label as coupled to said first binding partner and a second amount of said third binding partner coupled to the same label as said second binding partner and

said first amount of said third binding partner is not coupled to the same label as coupled to the second binding partner and said second amount of said third binding partner is not coupled to the same label as coupled to the first binding partner.

In addition such kits can also contain means for specific binding of the binding partners to their analytes, which may include buffers, blocking reagents and other reaction components known in the art. Kits for determination of nucleic acid analytes may also contain reaction components for nucleic acid amplification methods as described above. A PCR reaction kit could for example also contain a polymerase, buffers, nucleotide triphosphates and/ or primers.

The present invention is exemplified by the following examples:

Examples

*Example 1*

Multi-color analysis according to the present invention can be done using the Roche Cobas Taqman amplification/ detection technology and 5'-nuclease assay technology (EP 0543942 and US 5210015) building on "classical" fluorochrome compounds and "classical" resonance energy transfer principles. A Roche Cobas Taqman instrument is equipped with up to 4 filter pairs (see also EP 0953379, EP 0953837, US 6134000 and US 6084669). A putative indicator set could consist of 4 reporters and preferentially 1 or 2, optionally non-fluorescent, quenchers. Candidate compounds that span the spectral range set by the Cobas Taqman detector, i.e. ca. 420 nm - ca. 710 nm, may include:

- for quencher (Q) dyes with broad-banded absorption range in the red, for instance polymethin-cyanine dyes (e.g. n = 5, i.e. 4 conjugated olefinic entities building the mesomeric structure), or rhodamin derivatives at the flanks symmetrically fused to unsaturated N-heterocyclyc moieties that participate in the conjugated mesomeric $\pi$-$e^{(-)}$
- system, both preferentially as non-fluorescent nitrophenyl-derivatives
- for reporter (R) dyes fluorescent molecules like coumarine dyes, fluorescein-type dyes like FAM or chlorinated derivatives, rhodamin-type dyes, oxazines or bodipy-type compounds. Three of these would be assigned to target parameters, one for monitoring IC.

General requirements pertinent to candidate dyes include: good chemical stability in stock solution and also at use level as ingredients in mastermix reagents; stable when exposed to ambient light during synthesis, purification and kit manufacture; stable when exposed to multiple heating /cooling cycles (e.g. n = 60) in PCR; sufficient spectral overlap and efficient energy transfer between corresponding RQ resonance pairs; high extinction coefficients (i.e. absorptivity); and especially for reporters high quantum yield in aquous solutions and in the pH range employed for Taqman-PCR (e.g. pH 7-8,5).

The new concept allows the measuring of the distribution of crosstalk- and background-corrected normalized signals of 3 reporter dyes and dual combinations thereof across the corresponding 3 optical channels of Cobas Taqman instead of having a fixed assignment of a particular dye, representing a particular assay parameter, and a corresponding channel. The principle would provide for a complete resolution (via reporter R1, R2, R3, R1+2, R1+3, R2+3) of a positive result obtained with a 6-parameter-multiplex assay, the maximum complexity taken into consideration.

Theoretically, in cases of single-label use, 100% of the respective processed signal is found in a given channel; in cases of dual label use, processed signals would be distributed evenly across 2 corresponding channels (50% + 50%).

Discrimination of positive / negative pools (donations) could be done based on an optimized algorithm for determination of threshold cycles (ct values, i.e. the point on the reaction coordinate, where the fluorescent signal intensity rises significantly above background level), sensing the slope profile of signal-over-time curves.

Resolution of the kind of infection (assay parameter, i.e. kind of pathogen, yielding detectable product) could be done via analysis of channel-specific contributions to the total area under the signal/time curves (AUC), or to the total normalized plateau signal intensity. There is no firm dose-response correlation for criteria like these which, however, is not mandatory for qualitative assays as needed for blood screening.

A suitable 6-parameter (P1 to P6) multiplex assay could comprise the following probes (Q= quencher, R= reporter and $N_i$= one of the four nucleotides):

parameter 1 - probe = 5'-Q-$(Ni)_g$-R1-$(N_i)_h$-3'-$PO_4$ + 5'-Q-$(N_i)_g$-R2-$(Ni)_h$-3'-$PO_4$
<u>5 pmol each</u> /100 µl reaction mix

parameter 2 - probe = 5'-Q-$(N_i)_a$-R1-$(N_i)_b$-3'-$PO_4$
<u>10pmol</u> / 100 µl reaction mix

parameter 3 - probe = 5'-Q-$(N_i)_c$-R2-$(N_i)_d$-3'-$PO_4$
<u>10pmol</u> / 100 µl reaction mix

parameter 4 - probe = 5'-Q-$(N_i)_e$-R3-$(N_i)_f$-3'-$PO_4$
<u>10pmol</u> / 100 µl reaction mix

parameter 5 - probe = 5'-Q-$(N_i)_k$-R1-$(N_i)_l$-3'-$PO_4$ + 5'-Q-$(N_i)_k$-R3-$(N_i)_l$-3'-$PO_4$
<u>5 pmol each</u> / 100 µl reaction mix

parameter 6 - probe = 5'-Q-$(N_i)_n$-R2-$(N_i)_m$-3'-$PO_4$ + 5'-Q-$(N_i)_n$-R3-$(N_i)_m$-3'-$PO_4$
<u>5 pmol each</u> / 100 µl reaction mix

In other words:

in cases 2 - 4, ca. 1*$6^{12}$ molecules of uniformly labelled probe (same oligonucleotide / same reporter) would be introduced into PCR per parameter;

in cases 1, 5 and 6, again ca.1* $6^{12}$ molecules of labeled probe would be introduced into PCR per parameter, ca. ½* $6^{12}$ molecules with one sort of tag, and ½*$6^{12}$ molecules with the other sort of tag (same oligonucleotide / different reporters), which would be consumed statistically balanced;

in all case, 10 pmol of parameter-specific probe would be available.

Regarding the risk of ambiguous results, and focusing on the use of singular labels and dual combinations only, one may argue that ambiguous results would probably be extremely rare in the case of co-infections of 2 parameters indicated by single labeled probe, respectively, e.g. P2 ⇔ R1 + P4 ⇔ R3. The probability of either a 1:1 co-infection in terms of number of starting molecules and identical extraction / amplification / detection efficiencies yielding a 50:50 signal distribution into 2 channels, or of different numbers of starting molecules in conjunction with extraction / amplification / detection efficiencies that precisely counter-balance the differences in titre and again yield a 50:50 distribution, is considered negligibly low. Thus, an uneven distribution of >(>)50 : <(<)50 : 0 or <(<)50 : >(>)50 : 0 would result *(case*

*I),* and so be distinguishable from e.g. P5 ⇔ R1R3 indicated by an even distribution of 50 : 0 : 50.

Co-infection by 2 parameters indicated by 1:1 mixtures of 2 dual labeled probes *(case II),* e.g. P1 ⇔ R1R2 and P5 ⇔ R1R3, should always be evident due to signal distribution into 3 channels (e.g. ca. 50 : 25 : 25, comparable extraction / amplification / detection eficiencies assumed).

Co-infection by 2 parameters, one represented by 1 reporter and the other by two, should be recognized due to signal distribution into 3 channels, ≈ 33 : 33 : 33 (*case III,* e.g. R1R2 + R3), or yield a ≈75 : ≈25 : 0 pattern *(case IV,* e.g. R1R2 + R1), again assuming comparable extraction / amplification / detection efficiencies. For case II and III, the common feature would that there is no optical channel related to target with essentially "zero signal". In other words, the "zero+x" threshold to discriminate against "true zero" will be important. In *case IV,* probably the most critical one, resolution does depend on the overall efficiency of the assay, which determines whether the resulting distribution is substanbtially different from a 50 : 50 situation, or not. Most probably, additional mathematical criteria have to be taken into view in oder to resolve ambiguities, especially of the latter kind. Advantageously, real-time Taqman-PCR offers such "helper arithmetics". This topic is dealt with below in some more detail.

The rare occurrences of ambiguity, i.e. the event of a 50 : 50 signal - distribution occuring from co-infection can further minimized by assigning extreme rarely found, and less efficiently amplified parameters to single labeled probes, which might be complemented by considering geographical distributions.

A putative assignment of reporters (R = Label) and assays for the determination of HIV-1-M, HIV-1-O, HIV-2, HCV, HBV and HAV could be as follows:

| | | | | | | |
|---|---|---|---|---|---|---|
| P1 | ⇔ | HIV-1-M | ⇔ | R1/R2 | Amplification and | high |
| P2 | ⇔ | HIV-1-O | ⇔ | R1 | detection efficiency: | low |
| P3 | ⇔ | HIV-2 | ⇔ | R2 | | low |
| P4 | ⇔ | HCV | ⇔ | R3 | | high |
| P5 | ⇔ | HBV | ⇔ | R1/R3 | | high |
| P6 | ⇔ | HAV | ⇔ | R2 / R3 | | medium |

IC (internal control) ⇔ R4.

For safety reasons and especially with regard to a commercial assay one can also add an internal control, which for example may be detected by a probe coupled to a fourth label (R4).

Here, co-infection of HIV-1-O and HIV-2 should be very unlikely, both being very rare and geographically well separated (which, however, may change over the years), and the overall result would be "HIV positive" even in case of such an unexpected event.

On the other hand, co-infections of HN-1-O and HCV or HIV-2 and HCV should not result in an even distribution of 50 : 0 : 50 or 0 : 50 : 50, given the current amplification efficiencies (low for HIV-1-O and HIV-2), and not be mistaken for HBV or HAV single infections, respectively. Thus it is possible to use a standard four channel fluorimeter for conducting a six-parameter assay including an internal control being separately detected, which is for example used in a Roche Cobas TaqMan-instrument.

Thus, this concept for complete resolution of a 6 parameter multiplex assay in terms of assay parameter would necessitate moderate additional efforts compared to the present chemical system and instrument platform. Likewise, the requirements in terms of a more sophisticated analytical software are not very demanding, as shown by the considerations depicted below. In some detail, this will include:

- double logistic tracking for 3 probes (same oligonucleotide, different fluorescent tags)

- standardized procedure to establish signal outputs for individual reporters from single tagged probes and elaboration of normalization factors (taking into account impact from the sequence environment in the respective oligonucleotides, degree of purification as indicated by e.g. Em(R/Q) ratios, wavelength dependent energy contents and spectral sensitivity of CTM ASICS)

- elaboration of the most reliable output criterion or set of criteria to serve as 100% count, determination of the variance of distribution patterns, and setting of acceptance range borders for expected values

- development of a software algorithm for analysis of distribution of corrected normalized signals into 3 target channels that incorporates all the parameters mentioned above

*Example 2*

With regard to an assay protocol for automated test devices like the Roche Cobas TaqMan™ instrument a suitable process scheme is given below (see also Figure 1).

The availability of new reagents given, the principal data collecting and data processing scheme of an automated assay could be as follows:

| | | | |
|---|---|---|---|
| - | data collection: | | status: |
| 1. | dark mean (= instrument noise) | DM | already done |
| 2. | dark drift (= drift correction factor as f(t)) | DD | already done |
| 3. | cross-talk and preset correction factors | XT | already done |
| 4. | signal output normalization factors for reporters (= preset factors to correct for quantum yield, micro-environmental effects, spectral sensitivity, ...) | $NF_{Ri}$ | additional |
| 5. | background intensity (= chemical noise, accumulated from for all probes contained in the mix) | BG | already done |
| 6. | measured total signal over time | TFI | already done |

Preferably all steps are done separately for the optical channels 1, 2, 3, and 4 on the Cobas Taqman- instrument.

**-** data processing:

1) for each channel, and as a signal-over-time graph,
$\{TFI_{f(t)} - \{(DM * DD_{f(t)}) + BG]\}*XT* NF_{Ri}$ = <u>NFI</u> is caluclated, i.e. channel-specific normalized corrected fluorescence intensities

2) if channels 1, 2, 3 correspond to reporter dyes, and channel 4 to IC dye, then
$NFI_4$ = 100% IC response intensity
$NFI_{1+2+3}$ = 100% target response intensity,

3) which is to be analyzed in terms of distribution across these channels, i.e. x % in channel 1, y % in channel 2, and z % in channel 3.

The resulting signal distribution numbers are then compared to the pre-calculated values given above, and the identity of the pathogen detected is deduced from the correlation of optical channel-specific gain and corresponding reporter dyes, plus auxiliary factors.

Thus, in other words, the assay is not calibrated with respect to target titre (i.e. it is still a qualitative screening assay, yielding a pos/neg result as appropriate e.g. for screening applications in blood banks), but with respect to signal output of parameter-specific probes at a given concentration (to identify the cause for the positive result of a multiplex amplification).

The individual sample analysis could proceed as indicated in Figure 1.

To comprehend the logic of this processing algorithm, let's consider the underlying assay principles.

In 5'-nuclease technology (Taqman-PCR, the preferred embodiment for the invention set forth here) amplification and detection reactions, respectively, are closely interwoven. To this end, detection probes with 2 particular chemical modification are added to the PCR mastermix. One of these modifications is a fluorogenic reporter group (R, for instance a derivative of 6-carboxy-fluorescein) covalently attached to the backbone of the probe, the other one is a dye (for instance a polymethine-cyanine derivative) capable of absorbing the fluorescent light of the reporter and to quench it (quencher, Q). The quencher is typically attached to the probe backbone at the 5'-end, whereas the reporter is located within the oligo sequence, spaced from the quencher by a number of nucleotide building blocks. Probes bind to target nucleic acids (sense or anti-sense strand) close to the 3'-end of a primer (reverse or forward). As soon as primer has annealed to the target and DNA-polymerase gets bound to the primer : target hybrid, elongation starts. Due to the 5'-nuclease activity of the enzyme, simultaneously with copy strand synthesis the probe is cleaved as soon as the polymerase reaches the probe binding site, reporter and quencher get separated, and the fluorescent signal becomes measurable. This process is repeated with every cycle, and more and more fluorescent reporter is accumulated in solution until reagent depletion at the end of the reaction. So, in a signal-over-time plot, sigmoidal growth curves are generated. The point on the time axis, where the arbitrary fluorescent intensity (AFI, also called relative light units, RLU) can be significantly distinguished from background signal is called threshold cycle (ct). ct is a measure of analyte titre:

the smaller the ct-value, the higher the number of starting molecules and/or the better the extraction or amplification/ detection efficiency. ct values can be calculated by means of different mathematical operations. For example, cut-off approaches (average background signal intensity multiplied by a constant factor yields a cut-off signal intensity to discern negative from positive) can be used, or approaches where the location of the maximum of the first or second derivative of the signal-over-time curve (i.e. the steepness or slope profile) is sensed after curve fitting. The slope profile approach is, in contrast to the signal threshold approach, essentially independent of the background intensity level and, thus highly attractive for multiplex assays with cumulated background from all probes present in the reaction mixture.

Based on these principles, and according to the processing scheme depicted above, one would employ different means to generate the primary result (i.e. positive or negative?), and the secondary result (i.e. which kind of infection renders the sample positive?).

Compared to performing a multiplex assay with one common label, there is potential loss in sensitivity due to dilution of signal into several optical channels and, thus, reduced specific signal growth curves in particular channels. Therefore, in order to generate the primary result, one would optionally work with composite signal-over-time curves. By this means, the entire specific signal yield is collected into one signal-over-time curve which then is used to determine the threshold cycle (ct) and the principal status of the sample (pos. / neg.). Using the slope profile approach, the addition of background intensity would not be detrimental, and one could exploit the potential advantage of sensing the more pronounced growth characteristics of the specific signal gain.

On the other hand, for generation of secondary results, one would refer to the channel-specific distribution of normalized signal (NFI intensities), and deduce the kind of infection by comparing the resulting distribution patterns to the pre-calculated matrix of patterns given above. In addition, and in order to enhance the discriminatory power, also non-normalized *specific* signal intensities may be considered for analysis of ca. 50 : ca. 50 or ca. 75 : ca. 25 distributions of normalized signal, i.e. the really critical ones.

- In case of a *mono-infection* represented by a 1:1 mixture of probes, the resulting ratio of non-normalized, yet background-corrected signals R1 / R2 would be constant throughout the entire amplification/detection process, for instance 40 / 50. This must be precisely equivalent to the value of the normalization factor (0,80) obtained with equal amounts of purified dye, because both sorts of labelled probe (same oligonucleotide sequence!) would be cleaved at identical rates. Background (BG)-correction is necessary, since with a resolving 6-parameter-multiplex assay according to the concept presented here, each channel-specific background is made up from the signal output of 3 different probes tagged with the same label. Therefore, BG intensity is not only a function of the optical features of the label, but also of the structural features of the probe, i.e. linear or hairpin, or (more generally) by the degree of self-complementarity, and of the temperature during measurements which affects conformational equilibria of secondary structures.. So, secondary structures of unhybridized probe must be taken into view with respect to BG intensity, and auxiliary calculations have to be based on *specific* fluorescence intensities, SFI, with

$$SFI = TFI_{f(t)} - [(DM * DD_{f(t)}) + BG] * XT_i = AFI - BG.$$

  *Specific* signal, by contrast, is generated by way of 5'-nuclease activity of the polymerase depending on essentially perfect hybridization of probe to target, i.e. linearization of the probe oligonucleotide and, thus, essentially independent of probe structure. This pertains also to the truncated probe after having been cleaved and dissociated from the target strand due to the concomitant drop in Tm (melting temperature). Short internal hybrids in the residual probe oligomer should not be stable at the elevated temperatures used for annealing / elongation in PCR. After normalization, which compensates for differences in quantum yield or susceptibility to solvent effects, signal distribution in channel 1 and 2 would be 50 : 50. Finding NFI <u>*and*</u> SFI ratios as expected is double proof for mono-infection.

- In case of a *double infection* indicated via 2 specific probes labelled R1 and R2 *(case I),* respectively, it is very likely that the signal dynamics (i.e. growth as a function of time) is different for the 2 different assays, as either initial analyte titre, or overall assay efficiency, or both will probably be different. Thus, in addition to the probability of a > 50 : < 50 situation on the NFI level, also the SFI ratio of R1 / R2 must be different for rather early, central, and late phases of the reaction, i.e. the SFI ratio will be variable. The ratios will have a certain trend, and be different from the respective normalization factor obtained with equivalent mixtures of purified dye, preferably coupled to a model probe (to account for inevitable microenvironmental factors, e.g. linker, local charges), e.g. a $T_3$-R-$T_{16}$-oligonucleotide, which does not modulate signal output by its structural configuration.

- In case of a *double infection* represented by R1 + R1R2 *(case* IV) and substantially different overall recoveries for the 2 assays, a normalized signal distribution not very different from 50 : 50, e.g. 55 : 45, might be observed in channel 1 and 2. In terms of non-normalized signal intensities, however, the ratio R1 / R2 would be e.g. 44 / 50 (0,88), i.e. greater than the normalization factor. Thus, the double bias of "greater than expected" on both the normalized and non-normalized level, would indicate double infection. What is more, the ratio R1 / R2, e.g. traced e.g. via AUC, would most probably not be constant throughout the entire assay due to differing signal dynamics resulting from different amplification efficiencies, which will enhance discrimination.

- If, in case of double infection, both assays have ca. equal efficiency and start from either substantially different or ca. equal analyte titres, a 75 : 25 : 0 NFI pattern would be approximated, or even exceeded, with either a R1 + R2 or a R1 + R1R2 labelling *(case I OR case IV?)*. Here, discrimination between a *case IV* and a strongly *biased case I*-type co-infection may be achieved by means of absolute *specific* signal intensities (R1 intensity should be much lower in a *case I* situation) or absolute differences of channel-specific, optionally rectified, SFI values. Results might not completely unambiguous in some cases, yet.

In addition to this, composite AFI/t curves may be analysed concerning curve shape characteristics. In case of a mono-infection, one does expect a mono-sigmoidal curve with 1 point of inflection. In case of a double infection, however, it is most likely that the 2 growth curves generated from each of the 2 kind of nucleic acid targets extracted from the 2 infectious agents are not identical in terms of ct (i.e. point on the time axis where the curve bends upward and rises above background and drift level), in terms of shape, and specific signal intensity. Thus, a bi-sigmoidal curve shape may be generated, with 2 points of inflection. So, mono- or bi-sigmoidal curve shapes might be indicative of mono- or bi-infections present in the sample under inspection. In unfavourable cases, however, superposition of 2 or more curves may lead to irregular shapes with no clearcut maximum of slope, and with noise (i.e. the roughness of the curve) being increased. Still another possibility is a composite curve with 1 point of inflection, when 2 nearly identical curves are superposed.

To sum up, there are a number of mathematical means available to achieve resolution of a positive multiplex assay result in terms of the type in infection. These include, but are not limited to:

Basic means - reference NFI distribution patterns, and
type of deviation, either in situations with significant signal intensity in 2, or in 3, optical channels related to target. The accepted range of deviations from the reference pattern can be set by absolute numbers, % bands relative to the respective NFI value, based on precision data, or a combination of these.

Auxiliary means - analysis of NFI <u>and</u> SFI ratios pertinent to particular optical channels for common bias;
analysis of NFI or SFI ratios as a function of cycle number, i.e. dynamically along the reaction coordinate (constant or variable);
relationship of NFI ratios and corresponding absolute SFI values, or absolute SFI differences between any 2 optical channels (optionally rectified by relating to a standard dose difference), taking into account known assay-specific overall efficiencies (extraction / amplification / detection) for the different infectious agents under inspection;
analysis of composite AFI-over-time curve shapes (if applicable)

A putative application mode for this set of criteria is illustrated in Fig. 1 + 2.

What is more, in order to monitor the whole process, an artificial nucleic acid construct may be added to all samples, preferably packaged (armoured) in a modified virus particle, which is co-extracted and co-amplified with the natural target. This internal control (IC) features a unique probe binding region for an IC detection probe, which differs from target-specific probes by a different reporter group with distinguishable emission characteristics. Thus, IC signal can be discerned from target signal, and as the IC is known to be present in the sample, it functions as a monitoring agent. If there is no IC response, the respective reaction has to be considered invalid, and repeated.

Additional to the co-extracted/-amplified/-detected internal control (IC), added to each and every specimen to be processed (i.e. both unknowns and external controls or calibrators) and indicated by R4, external positive controls will be part of the assay. These are "knowns" which must be found positive, as opposed to "unknowns", and may be designed as mixed control samples that give rise to signal in all target related optical channels. Combined with the IC, utility of the entire optical system would be monitored then in addition to the functionality of the complete extraction/ amplification/detection process. For nucleic acid-based screening applications in blood banks, for instance, a highly sensitive, yet qualitative assay is required to identify each and every positive donation, as far as technically feasible. Therefore, titres of external postitive controls would be set in the lower range of concentration

*Example 3*

Based on the parameter list given in example 1 the signal distribution shown below is analyzed. For each of the 6 parameters under inspection, there is a reference pattern of signal distribution across the 3 optical channels assigned to target detection. Each reference pattern is complemented by a concomitant accepted range, for instance

| | Ch1 | Ch2 | Ch3 |
|---|---|---|---|
| P1 | 50 | 50 | 0 |
| P2 | 100 | 0 | 0 |
| P3 | 0 | 100 | 0 |
| P4 | 0 | 0 | 100 |
| P5 | 50 | 0 | 50 |
| P6 | 0 | 50 | 50 |

NFI reference pattern

| | Ch1 | Ch2 | Ch3 |
|---|---|---|---|
| P1 | 10 | 10 | 9,17 |
| P2 | 10 | 1,67 | 6,25 |
| P3 | 7,08 | 10 | 7,92 |
| P4 | 4,17 | 1,67 | 10 |
| P5 | 10 | 4,17 | 10 |
| P6 | 5,83 | 10 | 10 |

Putative 10% / 20% off set bands pertinent to 100% and 50% mean values, and 5 X multiples of near zero values, which translates into the following accepted ranges for the reference pattern

| | Ch1 | Ch2 | Ch3 |
|---|---|---|---|
| P1 | 40-60 | 40-60 | 0-9 |
| P2 | 90-100 | 0-2 | 0-6 |
| P3 | 0-7 | 90-100 | 0-8 |
| P4 | 0-4 | 0-2 | 90-100 |
| P5 | 40-60 | 0-8 | 40-60 |
| P6 | 0-8 | 40-60 | 40-60 |

Accepted ranges of deviation may be calculated, for example, as percent values (%), absolute numbers, multiples of simple standard deviation, or combinations thereof.

For each sample (parameter) the signal distribution in Channel 1 (reporter R1), Channel 2 (reporter R2) and Channel 3 (reporter R3) is given by 6 parallel simulated data sets based on 6-fold determinations each, as shown on the table (right side). On the left side the subsequent steps for evaluating the signal data are shown.

## Simulated distribution

| | Ch1 | Ch2 | Ch3 |
|---|---|---|---|
| **P1** | ***49,33*** | ***48,83*** | 1,83 |
| **P2** | ***98,42*** | 0,33 | 1,25 |
| **P3** | 1,42 | ***97,00*** | 1,58 |
| **P4** | 0,83 | 0,33 | ***98,83*** |
| **P5** | ***48,17*** | 1,67 | ***50,17*** |
| **P6** | 1,17 | ***50,33*** | ***48,50*** |

**mean values (arithmetic)**
***According to reference pattern***, deviated

| | Ch1 | Ch2 | Ch3 |
|---|---|---|---|
| **P1** | ***5,25*** | ***6,12*** | 5,17 |
| **P2** | ***4,69*** | 2,45 | 3,25 |
| **P3** | 2,75 | ***5,02*** | 3,34 |
| **P4** | 3,51 | 1,55 | ***4,81*** |
| **P5** | ***7,92*** | 6,75 | ***7,92*** |
| **P6** | 3,51 | ***6,48*** | ***7,29*** |

**threefold standard deviation**
***according to reference pattern***, deviated

| | Ch1 | Ch2 | Ch3 |
|---|---|---|---|
| **P1** | ***50*** | ***50*** | 0 |
| **P2** | ***100*** | 0 | 0 |
| **P3** | 0 | ***100*** | 0 |
| **P4** | 0 | 0 | ***100*** |
| **P5** | ***50*** | 0 | ***50*** |
| **P6** | 0 | ***50*** | ***50*** |

***NFI reference pattern***

| | Ch1 | Ch2 | Ch3 |
|---|---|---|---|
| **P1** | **10,00** | **10,00** | **9,17** |
| **P2** | **10,00** | **1,67** | **6,25** |
| **P3** | **7,08** | **10,00** | **7,92** |
| **P4** | **4,17** | **1,67** | **10,00** |
| **P5** | **10,00** | **8,33** | **10,00** |
| **P6** | **7,50** | **10,00** | **10,00** |

**10% / 20%-off-set bands pertinent to 100% and 50%-mean values, and factor 5 pertinent to 0% values i.e. accepted ranges for reference pattern**

Simulated data-set:

| | Ch1 | Ch2 | Ch3 | Total |
|---|---|---|---|---|
| P1 | 52 | 48 | 0 | 100 |
| | 49 | 48 | 3 | 100 |
| | 47 | 52 | 1 | 100 |
| | 50 | 46 | 4 | 100 |
| | 48 | 49 | 3 | 100 |
| | 50 | 50 | 0 | 100 |
| P2 | 98 | 0 | 2 | 100 |
| | 97,5 | 0 | 2,5 | 100 |
| | 99 | 0 | 1 | 100 |
| | 100 | 0 | 0 | 100 |
| | 96 | 2 | 2 | 100 |
| | 100 | 0 | 0 | 100 |
| P3 | 1 | 96 | 3 | 100 |
| | 2 | 96 | 2 | 100 |
| | 2 | 97 | 1 | 100 |
| | 1 | 99 | 0 | 100 |
| | 0 | 99 | 0 | 100 |
| | 2,5 | 95 | 2,5 | 100 |
| P4 | 0 | 0 | 100 | 100 |
| | 0 | 0 | 100 | 100 |
| | 3 | 1 | 96 | 100 |
| | 1 | 0 | 99 | 100 |
| | 0 | 0 | 100 | 100 |
| | 1 | 1 | 98 | 100 |
| P5 | 46 | 6 | 48 | 100 |
| | 50 | 1 | 49 | 100 |
| | 45 | 0 | 55 | 100 |
| | 52 | 0 | 48 | 100 |
| | 49 | 1 | 50 | 100 |
| | 47 | 2 | 51 | 100 |
| P6 | 0 | 50 | 50 | 100 |
| | 1 | 49 | 50 | 100 |
| | 1 | 53 | 46 | 100 |
| | 2 | 47 | 51 | 100 |
| | 3 | 52 | 45 | 100 |
| | 0 | 51 | 49 | 100 |

With regard to P5 a distribution of Ch1:Ch2:Ch3 of approximately 50:0:50 is found, with both mean values and scatter being within the accepted ranges. This would lead to the result "P5-positiv", which means monoinfection with HBV.

*Example 4*

As outlined above in the course of the principal considerations given in Examples 1 and 2, 50 : 50 distributions may not always be a valid indicator of mono-infections. Although it has to be mentioned that there are several applications where such ambigious results do not occur, in many other cases especially for diagnostic applications a further analysis often is desired. In order to allow a further analysis for example additional curve characteristics obtained in homogeneous amplification methods can be used. This is reflected in the scheme shown in Fig. 5, where additional curve characteristics like absolute SFI levels of the Ry dye and NFI trace features (NFI ratios constant / variable; early / late approximation of final distribution; bias <1 / > 1; etc.) are employed to resolve the infecting parameter(s).

It is evident from these examples that resolution should be significantly enhanced by analysing the real-time assay data dynamically along the reaction coordinate, instead of considering end-point conditions only. Especially for the samples S4 - S6, pure end-point analysis only would engender the risk of diagnosing mono-infections, while the comprehensive set of analytical tools allows for more precise assignments. In case of S5, for instance, the end-point NFI distribution mimicks HBV mono-infection as in Example 3, whereas by way of analysis of NFI distributions dynamically along the reaction coordinate, the false assignment "HBV mono-infection" can be rejected easily, and replaced by the correct assignment "Co-Infection, HIV-1-O + HCV", as HCV being amplified efficiently and represented by R3, gives rise to specific fluorescence intensity (SFI) much earlier than HIV-1-O, being amplified less efficiently, and represented by R1. Thus, the R1/R3 graph falls below the 50 : 50 baseline quickly, continues with positive bias, as R1 is produced with some delay, approximating the final distribution.

*Example 5*

Experimental data with HBV mono-infections

Genomic target (HBV subtype A), varying in dose from 25 - 5000 copies /ml, and internal control constructs were extracted from plasma samples using magnetic glass particle technology as described in PCT/EP00/11459. Eluted nucleic acids were specifically amplified and detected in homogeneous real-time Taqman-PCR on a COBAS TaqMan™ instrument operating under AmpliLink® version 2.1 (Roche Molecular Systems). For the basic studies reported here, HBV specific probe (code JW144) was applied @ 7,5 pmol/100 µl labelled with FAM ($\lambda_{EX} \approx 494$ nm; $\lambda_{EM} \approx 518$ nm), and @ 7,5 pmol/100 µl labelled with JA274 ($\lambda_{EX} \approx 580$ nm; $\lambda_{EM} \approx 609$ nm). Results were exported to MS Excel@ Version 7.0 for further data analysis according to the present invention (e.g. calculation of valid cycle range [SFI >> AFI-BG noise], of specific signal gain, normalised signal intensities, NFI ratios and NFI traces along the reaction coordinate, classification of NFI trace graphs into constant / offset / variable with positive slope / variable with negative slope, and for graphical representation of the results as well).

Homogeneous multiplex PCR reactions were performed under standard PCR conditions using primer pairs and probes specific for HIV-1M, HIV-10, HIV2, HCV and HBV.

As shown in Fig. 3, a-d, FAM and JA274 signals generated from one and the same biochemical reaction (i.e. extraction and amplification of HBV-DNA) behave in a coupled manner according to the concept of the present invention, i.e. swing in to the 50:50 distribution line as soon as SFI levels rise significantly above background. The higher the HBV dose, the earlier this is achieved, and remains constant until the end-point of the reaction. This is most noteworthy, since signal output as measured on the COBAS TaqMan™ is very different for FAM and JA274, resulting in a normalisation factor $NF_{(JA274/FAM)}$ of ca. 11,5 : 1. This in turn translates into enhanced analytical sensitivity of reactions indicated by JA274 compared with similar reaction indicated by FAM, i.e. at the lower end of detectable HBV doses, attaining to a 50 : 50 NFI distribution is critical using this dye pair ("worst case model")! Yet, upon translation of SFI into NFI using the experimentally established $NF_{(JA274/FAM)}$, these dyes - coupled by way of being generated from a common biochemical reaction - yield 50:50 distributions with remarkable precision (see also Fig. 4, b).

*Example 6*

Experimental data with different co-infections (type I & type IV)

Procedures and reagent sets were as described for Example 5, except that here, dependent on the targets extracted from co-infected samples, the probes specific for HBV, HCV, or HIV-1-O were consumed in the amplification / detection reaction. HBV probe was employed as a mixture of equal amounts of FAM- or JA274 labelled oligonucleotides, HCV probe was FAM labelled in total, and HIV-1-O probe was JA274 labelled in total. HBV + HCV positive plasma samples and HBV + HIV-1-O positive plasma samples are type IV co-infections, HCV + HIV-1-O positive is a type I co-infection. Note: for HIV-1-O, titres are given in X-fold dilutions of a culture supernatant; for all other parameters, precisely quantitated [cp/ml] and standardised material was available.

As can be seen from the plots in Fig. 4, a-d, NFI distribution *per se,* and NFI traces along the reaction coordinate are very different from those resulting from mono-infections. For instance,, in Fig. 4, a, the reactions # 1-12 feature low and approximately equivalent titres of HBV (amplifying very well; no reverse transcription step involved) and HCV (amplifying well, yet somewhat slower than HBV, via reverse transcription-PCR; HCV targets being not easy to access due to pronounced secondary structures). Consequently, soon after the on-set of the specific reaction $SFI_{(FAM)}$ and $SFI_{(JA274)}$ are produced via HBV reaction in considerable amounts, and $NFI_{(JA274/FAM)}$ ratios tend to approach the 50.50 distribution line (=1). As cycling progresses, however, HCV reacts with little delay, and additional $SFI_{(FAM)}$ is generated, pushing the $NFI_{(JA274/FAM)}$ ratio below the equity line with the graph having a negative slope. Reactions # 13-24 in Fig. 4, a feature low dose HBV infection plus a 100-fold excess of HCV dose, resulting in a shift of the valid cycle range towards earlier kick-off, an NFI $_{(JA274/FAM)}$ ratio graph that rushes down far below the equity line and then rises slowly with a positive slope as $SFI_{(FAM)}$ is generated from the high dose HCV reaction in advance of the "coupled" generation of $SFI_{(FAM)}$ and $SFI_{(JA274)}$ from the low dose HBV reaction, which gradually increases the ratio of NFI $_{(JA274/FAM)}$.

Thus, applying the criteria set forth in Example 2 (p. 25-26) in an ordered sequential manner as depicted in Fig. 1+2, the analysis would yield "Co-infection HBV + HCV". This holds true even for most reactions with a combination of high dose HBV and low dose HCV (high levels of $SFI_{(FAM)}$ and $SFI_{(JA274)}$ plus little additional $SFI_{(FAM)}$ alone, see Fig. 4, d) or from of high dose HBV and low dose HIV-1-O (high levels of $SFI_{(FAM)}$ and $SFI_{(JA274)}$ plus very little additional $SFI_{(JA274)}$ alone, see Fig. 4, c; reactions # 7-24). In a few cases, especially with the latter setting, both the shapes of NFI curves inspected visually and preliminary mathematical descriptors (NFI end-point ratios, NFI trace characteristics, absolute SFI levels, etc.) are quite close to those indicating mono-infection. This, however, can be improved by way of chemical (use of dyes with ca. similar emission intensities, i.e. NFI ratios more stable independent of target dose and concomitant level of absolute signal generation), mathematical (more sophisticated statistical tools applied to the analysis of primary growth and secondary NFI curves, respectively), as well as technical means (TC block and operations software configuration designed specifically for rapid cycling as with the TC profile used here; this helps to smooth the primary growth curves which in turn stabilises NFI ratio trace graphs).

What is more, Fig. 4, b (reactions # 13-24) and Fig. 4, c (reactions # 1-6) show examples of type-I co-infection, i.e. HCV plus HIV-1-O in this case, which results in a "non-coupled" generation of $SFI_{(FAM)}$ and $SFI_{(JA274)}$. Consequently, the NFI ratio trace graph runs, and remains, far below the equity line since HCV is amplified much better than HIV-1-O (a consequence, inter alia, of polymorphisms affecting the primer / probe binding sites). Moreover, also JA274 specific signal intensity generated from H1V-1-O alone are considerably lower than those generated from HBV mono-infections, which again is according to theory. This helps to further discriminate co-infection from mono-infection.

Generally speaking, type IV co-infections are more difficult to discriminate against mono-infections, especially when high doses of parameter 5 (generating high SFI levels for both R1 [Ry] and R3 [Rx]) is combined with very low doses of parameter 2 (generating only little additional R1), but in most cases, this is possible even using rather simple mathematical criteria as in this preliminary test system, and a "worst case model" dye pair. Combining 2 well amplifying targets in a type IV co-infection, e.g. parameter 5 [indicated by R1 + R3] and parameter 4 [indicated by R3] poses much less ambiguity, as substantial amounts of R3 intensity is added asynchronically and asymmetrically. Yet, if the relative abundance of parameter 4 versus parameter 5 becomes negligible, similar limits of resolution are observed. In contrast to type IV co-infection involving parameter 2 (and as expected), type I co-infections are discriminated quite easily against mono-infections indicated by the same, yet biochemically coupled dye pair. This becomes evident on the level of end-point NFI ratios and the NFI traces along the reaction coordinate as well, and also the significantly lower signal intensities of the R1 [Ry] dye. This is due to the fact that substantial R3 intensity is generated by parameter 4 even at low doses, while R1 intensity produced from cleavage of parameter 2-specifc probes is principally low and generated rather late during the course of 60 cycles. So, due to almost inevitable differences in either initial titre, or extraction / elution / amplification efficiency, it is most unlikely to yield a 50:50 distribution of signal throughout the reaction.

## Claims

1. A method for the determination of at least 3 nucleic acid analytes comprising the steps:

   a) Providing a mixture of a sample containing said at least 3 nucleic acid analytes or suspected of containing one or more of said nucleic acid analytes
   b) providing at least 3 different nucleic acid sequence specific probes
   c) amplifying said nucleic acid analytes
   d) choosing reaction conditions which promote the specific binding of said nucleic acid sequence specific probes to said amplified nucleic acid analytes, wherein

      - a first of said at least 3 nucleic acid sequence specific probes is specific for a first of said at least 3 nucleic acid analytes and is coupled to a first label,

- a second of said at least 3 nucleic acid sequence specific probes is specific for a second of said at least 3 nucleic acid analytes and is coupled to a second label, which label is separately detectable from the label coupled to said first nucleic acid sequence specific probe, and
- a third of said at least 3 nucleic acid sequence specific probes specific for a third of said at least 3 nucleic acid analytes whereby a first amount of said third nucleic acid sequence specific probe is coupled to the same label as coupled to said first nucleic acid sequence specific probe and a second amount of said third nucleic acid sequence specific probe is coupled to the same label as the second nucleic acid sequence specific probe and
said first amount of said third nucleic acid sequence specific probe is not coupled to the same label as coupled to the second nucleic acid sequence specific probe and said second amount of said third nucleic acid sequence specific probe is not coupled to the same label as coupled to the first nucleic acid sequence specific probe,

e) Detecting the signal intensities indicative for said first and second label,
f) Determining the nucleic acid analytes present in said sample using said signal intensities detected in step e).

**2.** Method of claim 1 **characterized in that** said nucleic acids were amplified using PCR.

**3.** Method of any of claims 1 to 2 **characterized in that** said labels were detected homogeneously.

**4.** Method of claim 3 **characterized in that** said labels were detected using fluorescence energy transfer.

**5.** Method of any of claims 1 to 4 **characterized in that** a first of said first, second and third nucleic acid sequence specific probe is specific for HIV, a second nucleic acid sequence specific probe is specific for HCV and a third nucleic acid sequence specific probe is specific for HBV.

**6.** Method of any of claims 1 to 4 **characterized in that** a first nucleic acid sequence specific probe is specific for HIV-1-M, a second nucleic acid sequence specific probe is specific for HIV-1-O, a third nucleic acid sequence specific probe is specific for HIV-2, a fourth nucleic acid sequence specific probe is specific for HCV, a fifth nucleic acid sequence specific probe is specific for HBV and a sixth nucleic acid sequence specific probe is specific for HAV.

**7.** A kit for the determination of at least 3 nucleic acid analytes containing in one or more containers

- a first nucleic acid sequence specific probe specific for a first nucleic acid analyte coupled to a first label,
- a second nucleic acid sequence specific probe specific for a second nucleic acid analyte coupled to a second label, which label is separately detectable from the label coupled to said first nucleic acid sequence specific probe,
- a third nucleic acid sequence specific probe specific for a third nucleic acid analyte, whereby a first amount of said third nucleic acid sequence specific probe is coupled to the same label as coupled to said first nucleic acid sequence specific probe and a second amount of said third nucleic acid sequence specific probe coupled to the same label as said second nucleic acid sequence specific probe, and
- reagents for nucleic acid amplification.

## Patentansprüche

**1.** Verfahren zur Bestimmung von wenigstens 3 Nukleinsäureanalyten, das die folgenden Schritte umfaßt:

a) Bereitstellen eines Gemischs einer Probe, die die wenigstens 3 Nukleinsäureanalyten enthält oder von der vermutet wird, daß sie einen oder mehrere der Nukleinsäureanalyten enthält
b) Bereitstellen von wenigstens 3 unterschiedlichen nukleinsäuresequenzspezifischen Sonden
c) Amplifizieren der Nukleinsäureanalyten
d) Wählen von Reaktionsbedingungen, die die spezifische Bindung der nukleinsäuresequenzspezifischen Sonden an die amplifizierten Nukleinsäureanalyten fördern, wobei

- eine erste der wenigstens 3 nukleinsäuresequenzspezifischen Sonden für einen ersten der wenigstens 3 Nukleinsäureanalyten spezifisch ist und an eine erste Markierung gekoppelt ist,
- eine zweite der wenigstens 3 nukleinsäuresequenzspezifischen Sonden für einen zweiten der wenigstens 3 Nukleinsäureanalyten spezifisch ist und an eine zweite Markierung gekoppelt ist, welche getrennt von der an die erste nukleinsäuresequenzspezifische Sonde gekoppelten Markierung nachweisbar ist, und
- eine dritte der wenigstens 3 nukleinsäuresequenzspezifischen Sonden für einen dritten der wenigstens 3 Nukleinsäureanalyten spezifisch, wodurch eine erste Menge der dritten nukleinsäuresequenzspezifischen Sonde an die gleiche Markierung wie die an die erste nukleinsäuresequenzspezifische Sonde gekoppelte Markierung gekoppelt ist und eine zweite Menge der dritten nukleinsäuresequenzspezifischen Sonde an die gleiche Markierung wie die zweite nukleinsäuresequenzspezifische Sonde gekoppelt ist und die erste Menge der dritten nukleinsäuresequenzspezifischen Sonde nicht an die gleiche Markierung wie die an die zweite nukleinsäuresequenzspezifische Sonde gekoppelte Markierung gekoppelt ist und die zweite Menge der dritten nukleinsäuresequenzspezifischen Sonde nicht an die gleiche Markierung wie die an die erste nukleinsäuresequenzspezifische Sonde gekoppelte Markierung gekoppelt ist,

e) Nachweisen der für die erste und die zweite Markierung bezeichnenden Signalintensitäten,
f) Bestimmen der in der Probe vorhandenen Nukleinsäureanalyten unter Verwendung der in Schritt e) nachgewiesenen Signalintensitäten.

**2.** Verfahren nach Anspruch 1, **dadurch gekennzeichnet, daß** die Nukleinsäuren mittels PCR amplifiziert wurden.

**3.** Verfahren nach einem der Ansprüche 1 bis 2, **dadurch gekennzeichnet, daß** die Markierungen homogen nachgewiesen wurden.

**4.** Verfahren nach Anspruch 3, **dadurch gekennzeichnet, daß** die Markierungen mit Fluoreszenzenergietransfer nachgewiesen wurden.

**5.** Verfahren nach einem der Ansprüche 1 bis 4, **dadurch gekennzeichnet, daß** eine erste der ersten, zweiten und dritten nukleinsäuresequenzspezifischen Sonde spezifisch für HIV, eine zweite nukleinsäuresequenzspezifische Sonde spezifisch für HCV und eine dritte nukleinsäuresequenzspezifische Sonde spezifisch für HBV ist.

**6.** Verfahren nach einem der Ansprüche 1 bis 4, **dadurch gekennzeichnet, daß** eine erste nukleinsäuresequenzspezifische Sonde spezifisch für HIV-1-M, eine zweite nukleinsäuresequenzspezifische Sonde spezifisch für HIV-1-O, eine dritte nukleinsäuresequenzspezifische Sonde spezifisch für HIV-2, eine vierte nukleinsäuresequenzspezifische Sonde spezifisch für HCV, eine fünfte nukleinsäuresequenzspezifische Sonde spezifisch für HBV und eine sechste nukleinsäuresequenzspezifische Sonde spezifisch für HAV ist.

**7.** Kit zur Bestimmung von wenigstens 3 Nukleinsäureanalyten, enthaltend in einem oder mehreren Behältern

- eine für einen ersten Nukleinsäureanalyten spezifische, an eine erste Markierung gekoppelte erste nukleinsäuresequenzspezifische Sonde,
- eine für einen zweiten Nukleinsäureanalyten spezifische, an eine zweite Markierung gekoppelte zweite nukleinsäuresequenzspezifische Sonde, wobei die Markierung getrennt von der an die erste nukleinsäuresequenzspezifische Sonde gekoppelten Markierung nachweisbar ist,
- eine für einen dritten Nukleinsäureanalyten spezifische dritte nukleinsäuresequenzspezifische Sonde, wodurch eine erste Menge der dritten nukleinsäuresequenzspezifischen Sonde an die gleiche Markierung wie die an die erste nukleinsäuresequenzspezifische Sonde gekoppelte Markierung gekoppelt ist und eine zweite Menge der an die gleiche Markierung wie die zweite nukleinsäuresequenzspezifische Sonde gekoppelten dritten nukleinsäuresequenzspezifischen Sonde, und
- Reagentien zur Nukleinsäureamplifikation.

## Revendications

**1.** Procédé pour la détermination d'au moins 3 d'analytes d'acide nucléique comprenant les étapes:

a) fournir un mélange d'un échantillon contenant lesdits au m oins 3 analytes d'acide nucléique ou soupçonné de contenir un ou plusieurs desdits analytes d'acide nucléique
b) fournir au moins 3 sondes différentes spécifiques pour des séquences d'acide nucléique
c) amplifier lesdits analytes d'acide nucléique
d) choisir des conditions de réaction qui favorisent la liaison spécifique desdites sondes spécifiques pour des séquences d'acide nucléique auxdits analytes d'acide nucléique amplifiés, dans lequel

- une première sonde parmi lesdites 3 sondes spécifiques pour des séquences d'acide nucléique est spécifique pour un premier analyte parmi lesdits au moins 3 analytes d'acide nucléique et est couplée à un premier marqueur,
- une deuxième sonde parmi lesdites au moins 3 sondes spécifiques pour des séquences d'acide nucléique est spécifique pour un deuxième analyte parmi lesdits au moins 3 analytes d'acide nucléique et est couplée à un premier marqueur, lequel marqueur est détectable séparément du marqueur couplé à ladite première sonde spécifique pour des séquences d'acide nucléique, et
- une troisième sonde parmi lesdites au moins 3 sondes spécifiques pour des séquences d'acide nucléique spécifique à un troisième analyte parmi lesdits au moins 3 analytes d'acide nucléique, à la suite de quoi une première quantité de ladite troisième sonde spécifique pour des séquences d'acide nucléique est couplée au même marqueur que celui couplé à ladite première sonde spécifique pour des séquences d'acide nucléique, et une deuxième quantité de ladite troisième sonde spécifique pour des séquences d'acide nucléique est couplée au même marqueur que la deuxième sonde spécifique pour des séquences d'acide nucléique, et

ladite première quantité de ladite troisième sonde spécifique pour des séquences d'acide nucléique n'est pas couplée au même marqueur que celle couplée à la deuxième sonde spécifique pour des séquences d'acide nucléique, et ladite deuxième quantité de ladite troisième sonde spécifique pour des séquences d'acide nucléique n'est pas couplée a u même marqueur que la première sonde spécifique pour des séquences d'acide nucléique,

e) détecter les intensités de signal indicatives pour ledit premier et deuxième marqueurs,
f) déterminer les analytes d'acide nucléique présents dans ledit échantillon en employant lesdites intensités de signal détectées dans l'étape e).

**2.** Procédé selon la revendication 1, **caractérisé en ce que** lesdits acides nucléiques ont été amplifiés en employant la PCR.

**3.** Procédé selon l'une quelconque des revendications 1 à 2, **caractérisé en ce que** lesdits marqueurs ont été détectés de façon homogène.

**4.** Procédé selon la revendication 3, **caractérisé en ce que** lesdits marqueurs ont été détectés en employant un transfert d'énergie de fluorescence.

**5.** Procédé selon l'une quelconque des revendications 1 à 4, **caractérisé en ce qu'**une première sonde parmi lesdites première, deuxième, et troisième sondes spécifiques pour des séquences d'acide nucléique est spécifique au HIV, une deuxième sonde spécifique pour des séquences d'acide nucléique est spécifique au HCV et une troisième sonde spécifique pour des séquences d'acide nucléique est spécifique au HBV.

**6.** Procédé selon l'une quelconque des revendications 1 à 4, **caractérisé en ce qu'**une première sonde spécifique pour des séquences d'acide nucléique est spécifique au HIV-1-M, une deuxième sonde spécifique pour des séquences d'acide nucléique est sspécifique au HIV-1-O, u ne troisième sonde spécifique pour des séquences d'acide nucléique est spécifique au HIV-2, une quatrième sonde spécifique pour des séquences d'acide nucléique est spécifique au HCV, une cinquième sonde spécifique pour des séquences d'acide nucléique est spécifique au HBV et une sixième sonde spécifique pour des séquences d'acide nucléique est spécifique au HAV.

**7.** Nécessaire pour la détermination d'au moins 3 analytes d'acide nucléique contenant dans un ou plusieurs récipients

- une première sonde spécifique pour des séquences d'acide nucléique spécifique à un premier analyte à acide nucléique couplé à un premier marqueur,
- une deuxième sonde spécifique pour des séquences d'acide nucléique spécifique à un deuxième analyte d'acides nucléiques couplée à un deuxième marqueur, lequel marqueur est détectable séparément du marqueur couplé à ladite première sonde spécifique pour des séquences d'acide nucléique,
- une troisième sonde spécifique pour des séquences d'acide nucléique spécifique à un troisième analyte à acide nucléique, à la suite de quoi une première quantité de ladite troisième sonde spécifique pour des séquences d'acide nucléique est couplée au même marqueur que ladite première sonde spécifique pour des séquences d'acide nucléique et une deuxième quantité de ladite troisième sonde spécifique pour des séquences d'acide nucléique couplée au même marqueur que ladite deuxième sonde spécifique pour des séquences d'acide nucléique, et

- des réactifs pour une amplification d'acides nucléiques.

Fig 1

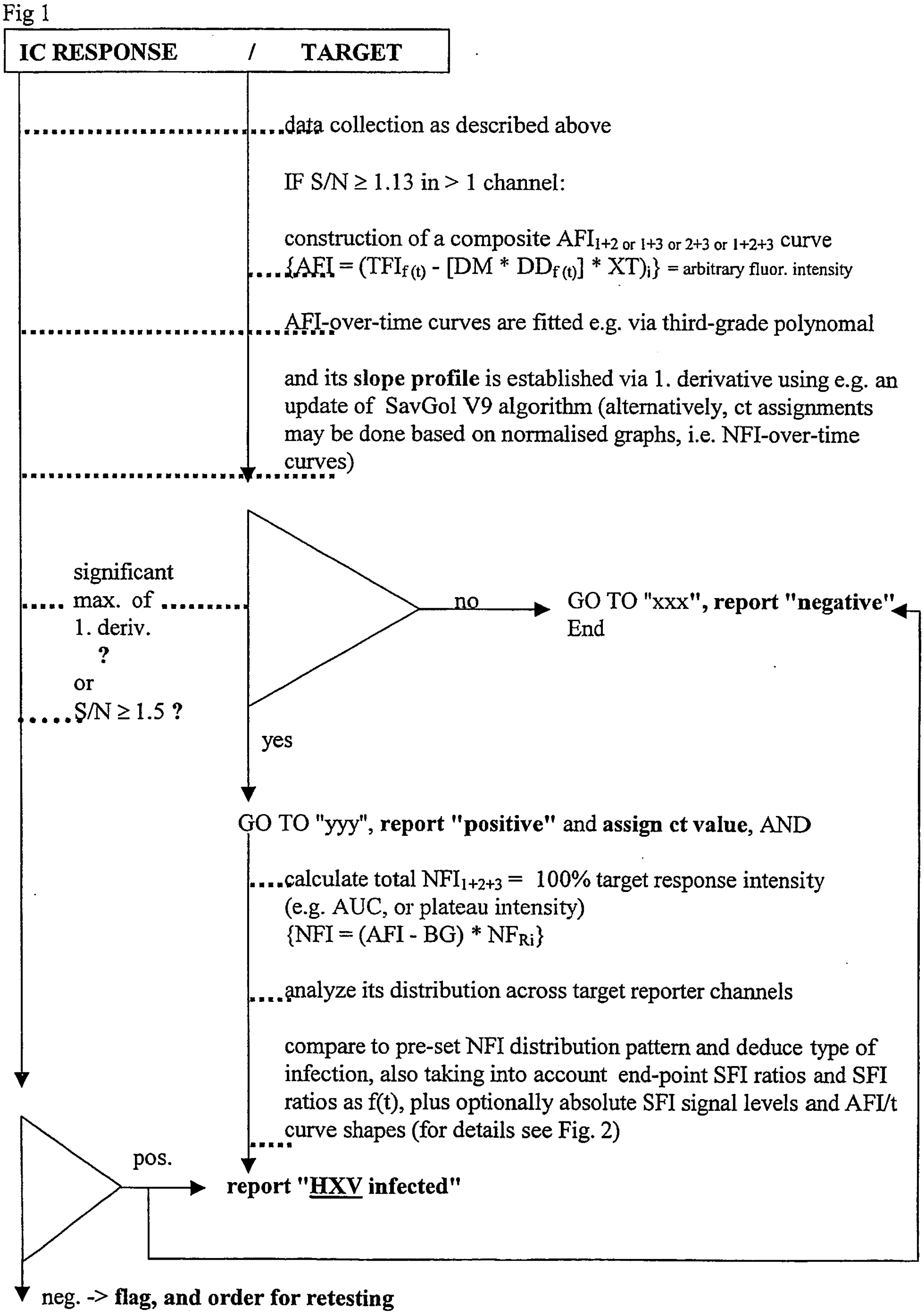
IC RESPONSE / TARGET
data collection as described above
IF S/N ≥ 1.13 in > 1 channel:
construction of a composite $AFI_{1+2 \text{ or } 1+3 \text{ or } 2+3 \text{ or } 1+2+3}$ curve
$\{AFI = (TFI_{f(t)} - [DM * DD_{f(t)}] * XT)_i\}$ = arbitrary fluor. intensity
AFI-over-time curves are fitted e.g. via third-grade polynomal
and its **slope profile** is established via 1. derivative using e.g. an update of SavGol V9 algorithm (alternatively, ct assignments may be done based on normalised graphs, i.e. NFI-over-time curves)
significant max. of 1. deriv. ?
or
S/N ≥ 1.5 ?
no
GO TO "xxx", **report "negative"**
End
yes
GO TO "yyy", **report "positive"** and **assign ct value**, AND
calculate total $NFI_{1+2+3}$ = 100% target response intensity
(e.g. AUC, or plateau intensity)
$\{NFI = (AFI - BG) * NF_{Ri}\}$
analyze its distribution across target reporter channels
compare to pre-set NFI distribution pattern and deduce type of infection, also taking into account end-point SFI ratios and SFI ratios as f(t), plus optionally absolute SFI signal levels and AFI/t curve shapes (for details see Fig. 2)
pos.
**report "HXV infected"**
neg. -> **flag, and order for retesting**

Fig. 2: **Deconvolution scheme for signal distribution analysis according to the present invention for an assay with the primary result "positive"**

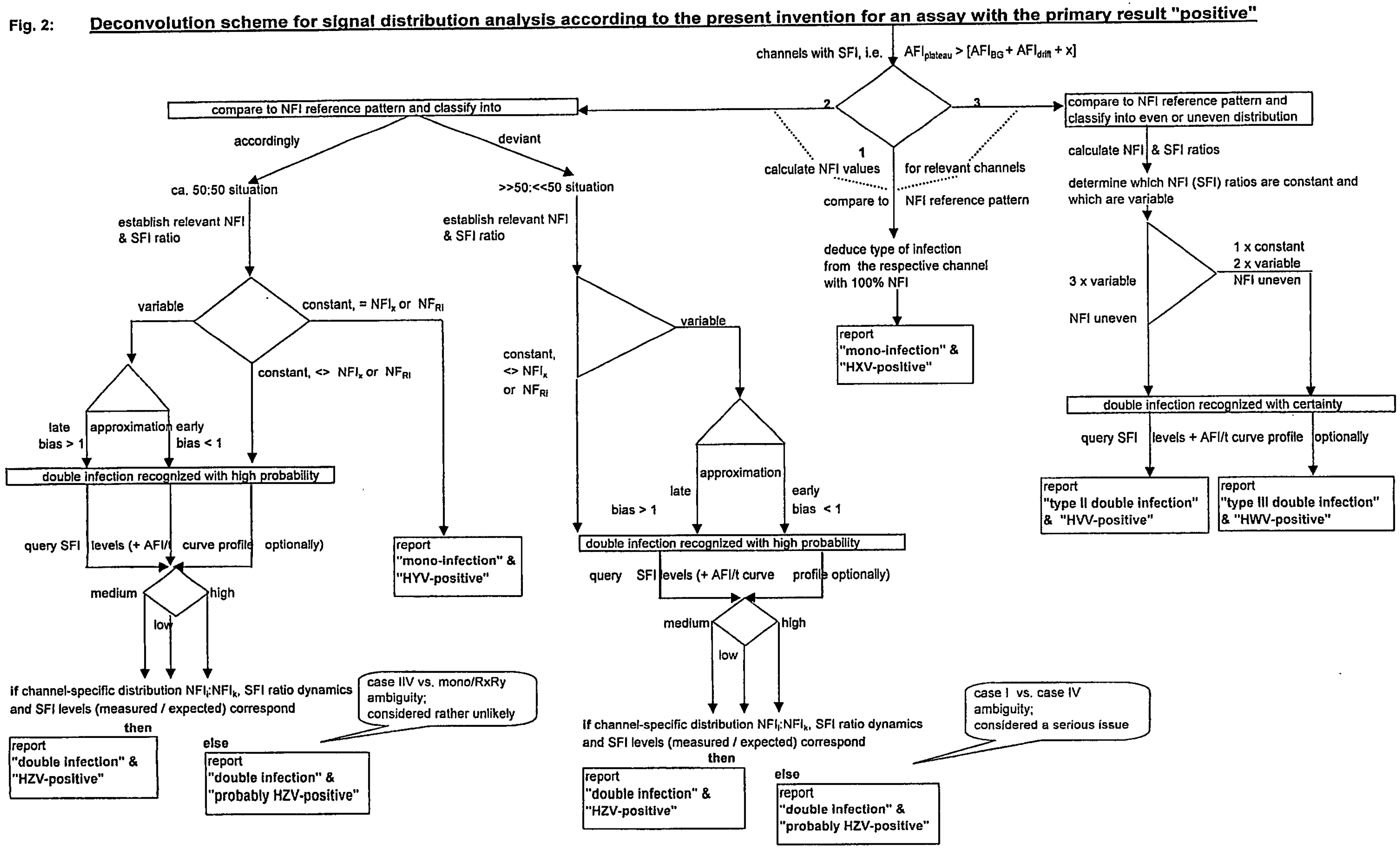

Fig 3a: NFI ratios (Ry / Rx) as f(t)

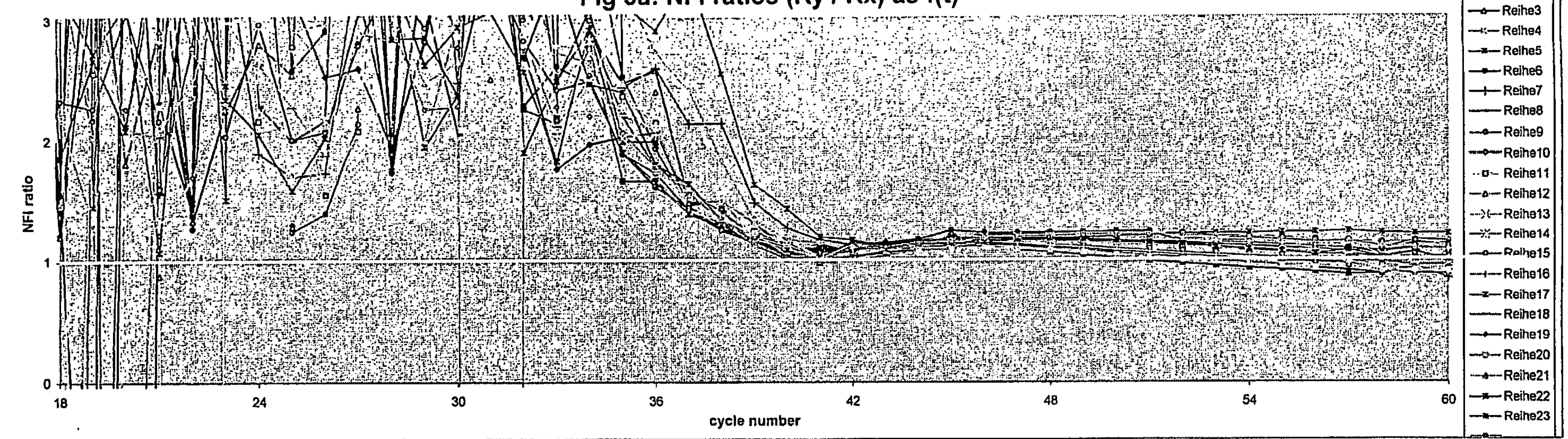


| Rx = | FAM | Ry = | JA274 | [cp/ml] | analyte nucleic acid | | SFI (Ry) | valid range (SFI ~> AFI) for reaction # | = | min | [cycles] | max | NFI ratio(55-60) | / trace |
|---|---|---|---|---|---|---|---|---|---|---|---|---|---|---|
| | | | | 25 | HBV | extracted | 831 | 1 | | 41 | - | 60 | 1,12 | constant |
| Normalization factor = | | 11,40 | | 25 | HBV | extracted | 554 | 2 | | 40 | - | 60 | 0,91 | constant |
| | => | | | 25 | HBV | extracted | 528 | 3 | | 41 | - | 60 | 1,08 | constant |
| Ry/NF: Rx = 50 : 50 distribution of NFI | | | | 25 | HBV | extracted | 674 | 4 | | 41 | - | 60 | 1,20 | constant |
| to be expected in case of coupled | | | | 25 | HBV | extracted | 506 | 5 | | 40 | - | 60 | 1,00 | constant |
| signal generation | | | | 25 | HBV | extracted | 794 | 6 | | 40 | - | 60 | 1,10 | constant |
| | => | NFI ratio = 1,0 | | 25 | HBV | extracted | 230 | 7 | | 43 | - | 60 | 0,91 | constant |
| else: | 85 : 15 | 5,67 | | 25 | HBV | extracted | 527 | 8 | | 40 | - | 60 | 1,04 | constant |
| | 80 : 20 | 4,00 | | 25 | HBV | extracted | 406 | 9 | | 39 | - | 60 | 1,15 | constant |
| | 75 : 25 | 3,00 | | 25 | HBV | extracted | 552 | 10 | | 40 | - | 60 | 1,10 | constant |
| | 70 : 30 | 2,33 | | 25 | HBV | extracted | 474 | 11 | | 40 | - | 60 | 0,95 | constant |
| | 65 : 35 | 1,86 | | 25 | HBV | extracted | 602 | 12 | | 40 | - | 60 | 1,16 | constant |
| | 60 : 40 | 1,50 | | 25 | HBV | extracted | 772 | 13 | | 40 | - | 60 | 1,04 | constant |
| | 55 : 45 | 1,22 | | 25 | HBV | extracted | 451 | 14 | | 41 | - | 60 | 0,98 | constant |
| | 53 : 47 | 1,13 | | 25 | HBV | extracted | 589 | 15 | | 40 | - | 60 | 0,94 | constant |
| | | | | 25 | HBV | extracted | 463 | 16 | | 40 | - | 60 | 0,95 | constant |
| | 47 : 53 | 0,89 | | 25 | HBV | extracted | 610 | 17 | | 40 | - | 60 | 1,00 | constant |
| | 45 : 55 | 0,82 | | 25 | HBV | extracted | 593 | 18 | | 41 | - | 60 | 1,01 | constant |
| | 40 : 60 | 0,67 | | 25 | HBV | extracted | 501 | 19 | | 42 | - | 60 | 0,92 | constant |
| | 35 : 65 | 0,54 | | 25 | HBV | extracted | 525 | 20 | | 41 | - | 60 | 0,93 | constant |
| | 30 : 70 | 0,43 | | 25 | HBV | extracted | 732 | 21 | | 40 | - | 60 | 1,07 | constant |
| | 25 : 75 | 0,33 | | 25 | HBV | extracted | 663 | 22 | | 40 | - | 60 | 1,03 | constant |
| | 20 : 80 | 0,25 | | 25 | HBV | extracted | 541 | 23 | | 43 | - | 60 | 1,22 | constant |
| | 15 : 85 | 0,18 | | 25 | HBV | extracted | 839 | 24 | | 40 | - | 60 | 1,12 | constant |

Fig 3b: NFI ratios (Ry / Rx) as f(t)

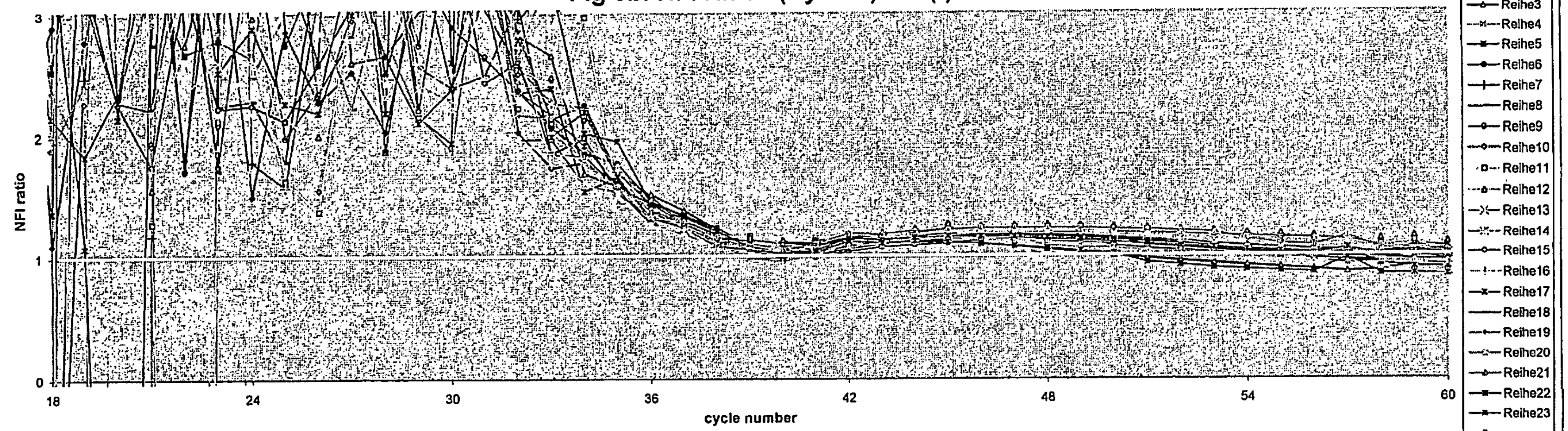


| Rx = | FAM | Ry = JA274 | [cp/ml] | analyte nucleic acid | | SFI (Ry) | valid range (SFI -> AFI) for reaction # | = | min | [cycles] | max | NFI ratio (55-60) / | trace |
|---|---|---|---|---|---|---|---|---|---|---|---|---|---|
| | | | 100 | HBV | extracted | 759 | 1 | | 38 | - | 60 | 1,00 | constant |
| Normalization factor = | | 11,40 | 100 | HBV | extracted | 786 | 2 | | 38 | - | 60 | 1,00 | constant |
| => | | | 100 | HBV | extracted | 606 | 3 | | 39 | - | 60 | 0,90 | constant |
| Ry/NF: Rx = 50 : 50 distribution of NFI | | | 100 | HBV | extracted | 645 | 4 | | 38 | - | 60 | 1,05 | constant |
| to be expected in case of coupled | | | 100 | HBV | extracted | 711 | 5 | | 39 | - | 60 | 1,03 | constant |
| signal generation | | | 100 | HBV | extracted | 714 | 6 | | 38 | - | 60 | 0,99 | constant |
| => | | NFI ratio = 1,0 | 100 | HBV | extracted | 598 | 7 | | 38 | - | 60 | 1,04 | constant |
| else: | 85 : 15 | 5,67 | 100 | HBV | extracted | 664 | 8 | | 37 | - | 60 | 1,03 | constant |
| | 80 : 20 | 4,00 | 100 | HBV | extracted | 631 | 9 | | 38 | - | 60 | 1,01 | constant |
| | 75 : 25 | 3,00 | 100 | HBV | extracted | 606 | 10 | | 38 | - | 60 | 1,09 | constant |
| | 70 : 30 | 2,33 | 100 | HBV | extracted | 704 | 11 | | 38 | - | 60 | 1,11 | constant |
| | 65 : 35 | 1,86 | 100 | HBV | extracted | 641 | 12 | | 38 | - | 60 | 1,16 | bordeerline |
| | 60 : 40 | 1,50 | 100 | HBV | extracted | 839 | 13 | | 38 | - | 60 | 1,04 | constant |
| | 55 : 45 | 1,22 | 100 | HBV | extracted | 687 | 14 | | 38 | - | 60 | 0,93 | constant |
| | 53 : 47 | 1,13 | 100 | HBV | extracted | 734 | 15 | | 39 | - | 60 | 1,01 | constant |
| | | | 100 | HBV | extracted | 749 | 16 | | 38 | - | 60 | 0,99 | constant |
| | 47 : 53 | 0,89 | 100 | HBV | extracted | 916 | 17 | | 38 | - | 60 | 0,98 | constant |
| | 45 : 55 | 0,82 | 100 | HBV | extracted | 753 | 18 | | 38 | - | 60 | 1,11 | constant |
| | 40 : 60 | 0,67 | 100 | HBV | extracted | 855 | 19 | | 38 | - | 60 | 0,99 | constant |
| | 35 : 65 | 0,54 | 100 | HBV | extracted | 780 | 20 | | 38 | - | 60 | 1,04 | constant |
| | 30 : 70 | 0,43 | 100 | HBV | extracted | 748 | 21 | | 39 | - | 60 | 0,99 | constant |
| | 25 : 75 | 0,33 | 100 | HBV | extracted | 762 | 22 | | 39 | - | 60 | 0,89 | constant |
| | 20 : 80 | 0,25 | 100 | HBV | extracted | 706 | 23 | | 38 | - | 60 | 1,02 | constant |
| | 15 : 85 | 0,18 | 100 | HBV | extracted | 757 | 24 | | 38 | - | 60 | 1,09 | constant |

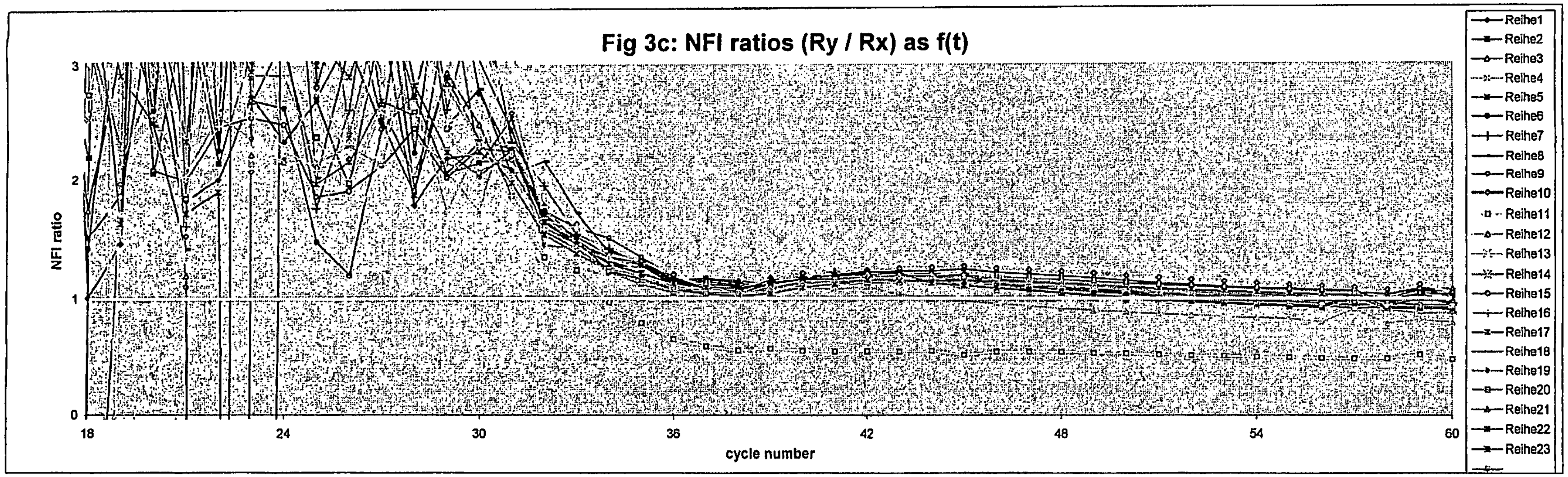


| Rx = | FAM | Ry = JA274 | [cp/ml] | analyte nucleic acid | | SFI (Ry) | valid range (SFI -> AFI) for reaction # | = | min | [cycles] | max | NFI ratio (55-60) | / trace |
|---|---|---|---|---|---|---|---|---|---|---|---|---|---|
| | | | 500 | HBV | extracted | 786 | 1 | | 36 | - | 60 | 0,98 | constant |
| Normalization factor = | | 11,40 | 500 | HBV | extracted | 918 | 2 | | 36 | - | 60 | 1,01 | constant |
| | => | | 500 | HBV | extracted | 937 | 3 | | 36 | - | 60 | 1,03 | constant |
| Ry/NF : Rx = 50 : 50 distribution of NFI | | | 500 | HBV | extracted | 782 | 4 | | 36 | - | 60 | 0,97 | constant |
| to be expected in case of coupled | | | 500 | HBV | extracted | 731 | 5 | | 36 | - | 60 | 0,96 | constant |
| signal generation | | | 500 | HBV | extracted | 823 | 6 | | 36 | - | 60 | 0,98 | constant |
| | => | NFI ratio = 1,0 | 500 | HBV | extracted | 690 | 7 | | 36 | - | 60 | 0,99 | constant |
| else: | 85 : 15 | 5,67 | 500 | HBV | extracted | 648 | 8 | | 36 | - | 60 | 1,00 | constant |
| | 80 : 20 | 4,00 | 500 | HBV | extracted | 672 | 9 | | 35 | - | 60 | 1,06 | constant |
| | 75 : 25 | 3,00 | 500 | HBV | extracted | 616 | 10 | | 35 | - | 60 | 1,10 | constant |
| | 70 : 30 | 2,33 | 500 | HBV | extracted | 148 | 11 | | 36 | - | 60 | 0,49 | var. / m- |
| | 65 : 35 | 1,86 | 500 | HBV | extracted | 661 | 12 | | 35 | - | 60 | 1,02 | constant |
| | 60 : 40 | 1,50 | 500 | HBV | extracted | 860 | 13 | | 36 | - | 60 | 0,95 | constant |
| | 55 : 45 | 1,22 | 500 | HBV | extracted | 1005 | 14 | | 35 | - | 60 | 0,97 | constant |
| | 53 : 47 | 1,13 | 500 | HBV | extracted | 903 | 15 | | 35 | - | 60 | 1,01 | constant |
| | | | 500 | HBV | extracted | 904 | 16 | | 35 | - | 60 | 0,99 | constant |
| | 47 : 53 | 0,89 | 500 | HBV | extracted | 856 | 17 | | 35 | - | 60 | 0,98 | constant |
| | 45 : 55 | 0,82 | 500 | HBV | extracted | 952 | 18 | | 36 | - | 60 | 0,95 | constant |
| | 40 : 60 | 0,67 | 500 | HBV | extracted | 767 | 19 | | 36 | - | 60 | 0,93 | constant |
| | 35 : 65 | 0,54 | 500 | HBV | extracted | 847 | 20 | | 36 | - | 60 | 0,93 | constant |
| | 30 : 70 | 0,43 | 500 | HBV | extracted | 698 | 21 | | 35 | - | 60 | 0,82 | constant |
| | 25 : 75 | 0,33 | 500 | HBV | extracted | 892 | 22 | | 35 | - | 60 | 0,95 | constant |
| | 20 : 80 | 0,25 | 500 | HBV | extracted | 855 | 23 | | 36 | - | 60 | 0,93 | constant |
| | 15 : 85 | 0,18 | 500 | HBV | extracted | 813 | 24 | | 35 | - | 60 | 0,96 | constant |

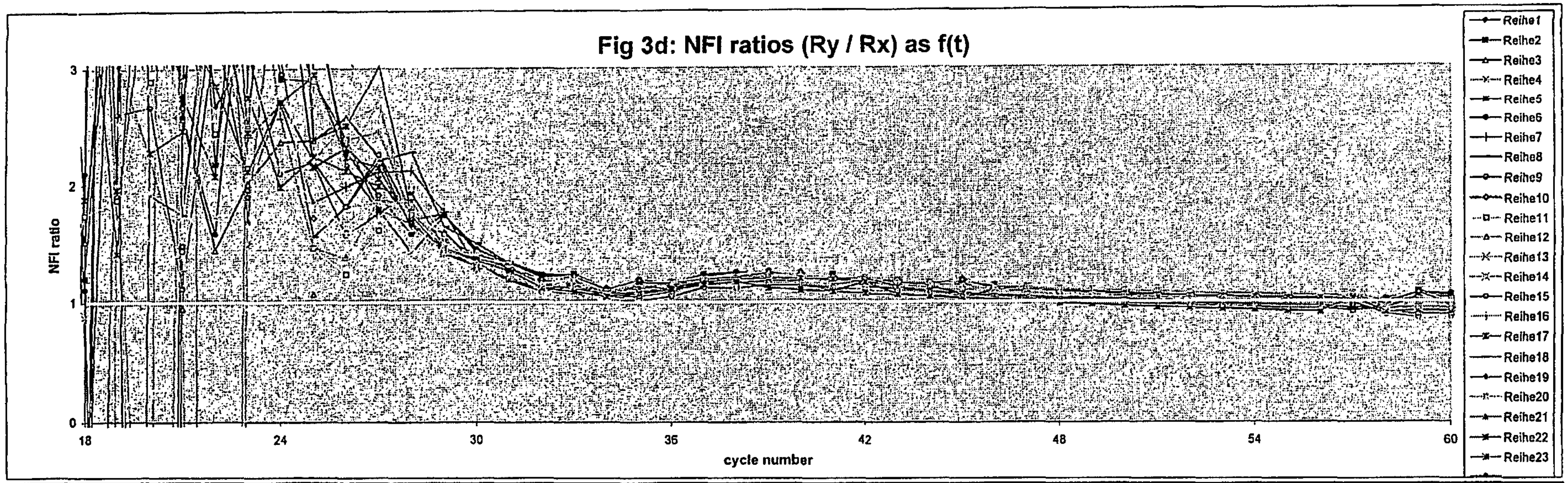

Fig 3d: NFI ratios (Ry / Rx) as f(t)

| Rx = | FAM | Ry = | JA274 | [cp/ml] | analyte nucleic acid | | SFI (Ry) | valid range (SFI --> AFI) for reaction # | = | min | [cycles] | max | NFI ratio (55-60) / trace | |
|---|---|---|---|---|---|---|---|---|---|---|---|---|---|---|
| | | | | 5000 | HBV | extracted | 741 | 1 | | 32 | - | 60 | 1,04 | constant |
| Normalization factor = | | 11,40 | | 5000 | HBV | extracted | 856 | 2 | | 32 | - | 60 | 0,99 | constant |
| => | | | | 5000 | HBV | extracted | 811 | 3 | | 32 | - | 60 | 0,97 | constant |
| Ry/NF : Rx = 50 : 50 distribution of NFI | | | | 5000 | HBV | extracted | 748 | 4 | | 32 | - | 60 | 1,02 | constant |
| to be expected in case of coupled | | | | 5000 | HBV | extracted | 802 | 5 | | 32 | - | 60 | 0,99 | constant |
| signal generation | | | | 5000 | HBV | extracted | 894 | 6 | | 32 | - | 60 | 0,99 | constant |
| => | | NFI ratio = 1,0 | | 5000 | HBV | extracted | 670 | 7 | | 32 | - | 60 | 1,02 | constant |
| else: | 85 : 15 | 5,67 | | 5000 | HBV | extracted | 735 | 8 | | 32 | - | 60 | 1,01 | constant |
| | 80 : 20 | 4,00 | | 5000 | HBV | extracted | 765 | 9 | | 32 | - | 60 | 1,02 | constant |
| | 75 : 25 | 3,00 | | 5000 | HBV | extracted | 837 | 10 | | 32 | - | 60 | 0,99 | constant |
| | 70 : 30 | 2,33 | | 5000 | HBV | extracted | 703 | 11 | | 31 | - | 60 | 1,03 | constant |
| | 65 : 35 | 1,86 | | 5000 | HBV | extracted | 701 | 12 | | 31 | - | 60 | 1,03 | constant |
| | 60 : 40 | 1,50 | | 5000 | HBV | extracted | 934 | 13 | | 32 | - | 60 | 0,96 | constant |
| | 55 : 45 | 1,22 | | 5000 | HBV | extracted | 845 | 14 | | 32 | - | 60 | 0,99 | constant |
| | 53 : 47 | 1,13 | | 5000 | HBV | extracted | 859 | 15 | | 32 | - | 60 | 0,94 | constant |
| | | | | 5000 | HBV | extracted | 813 | 16 | | 32 | - | 60 | 0,94 | constant |
| | 47 : 53 | 0,89 | | 5000 | HBV | extracted | 962 | 17 | | 32 | - | 60 | 0,96 | constant |
| | 45 : 55 | 0,82 | | 5000 | HBV | extracted | 827 | 18 | | 32 | - | 60 | 0,99 | constant |
| | 40 : 60 | 0,67 | | 5000 | HBV | extracted | 910 | 19 | | 32 | - | 60 | 1,01 | constant |
| | 35 : 65 | 0,54 | | 5000 | HBV | extracted | 870 | 20 | | 31 | - | 60 | 0,97 | constant |
| | 30 : 70 | 0,43 | | 5000 | HBV | extracted | 845 | 21 | | 32 | - | 60 | 0,93 | constant |
| | 25 : 75 | 0,33 | | 5000 | HBV | extracted | 957 | 22 | | 32 | - | 60 | 0,93 | constant |
| | 20 : 80 | 0,25 | | 5000 | HBV | extracted | 847 | 23 | | 32 | - | 60 | 0,96 | constant |
| | 15 : 85 | 0,18 | | 5000 | HBV | extracted | 902 | 24 | | 32 | - | 60 | 0,96 | constant |

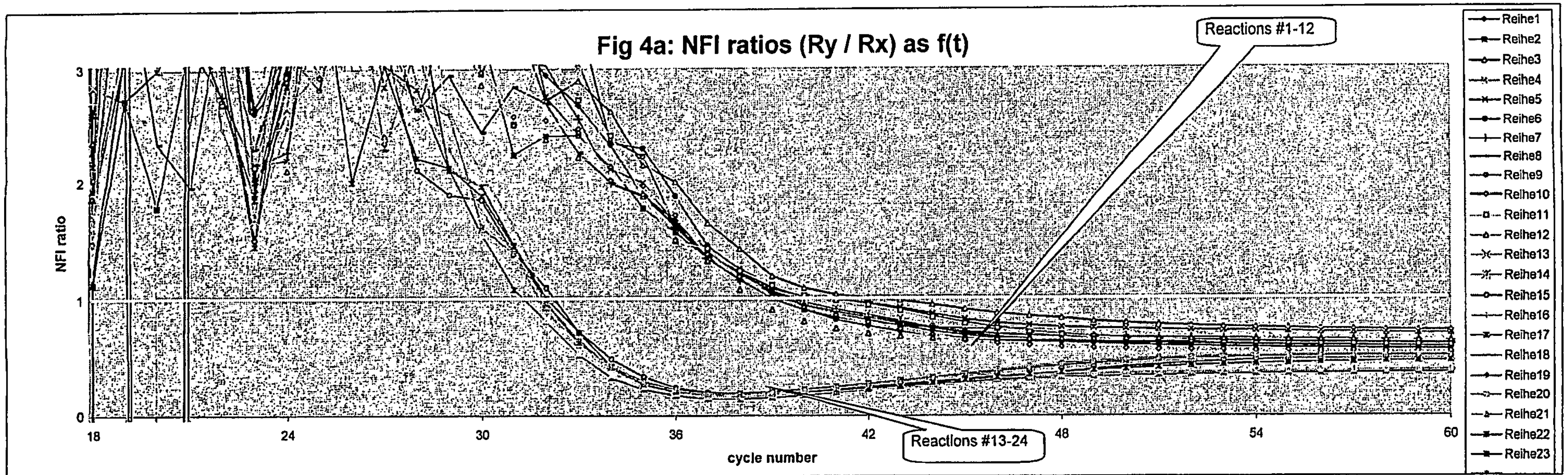


Fig 4a: NFI ratios (Ry / Rx) as f(t)

| Rx = | FAM | Ry = | JA274 | [cp/ml] | analyte nucleic acid | | SFI (Ry) | valid range (SFI -> AFI) for reaction # = | min | [cycles] | max | NFI ratio (55-60) / trace | |
|---|---|---|---|---|---|---|---|---|---|---|---|---|---|
| | | | | 50/50 | HBV/HCV | extracted | 755 | 1 | 39 | - | 60 | 0,59 | var. / m- |
| Normalization factor = | | 11,40 | | 50/50 | HBV/HCV | extracted | 671 | 2 | 38 | - | 60 | 0,71 | var. / m- |
| => | | | | 50/50 | HBV/HCV | extracted | 555 | 3 | 38 | - | 60 | 0,74 | var. / m- |
| Ry/NF: Rx = 50 : 50 distribution of NFI | | | | 50/50 | HBV/HCV | extracted | 687 | 4 | 38 | - | 60 | 0,72 | var. / m- |
| to be expected in case of coupled | | | | 50/50 | HBV/HCV | extracted | 704 | 5 | 39 | - | 60 | 0,71 | var. / m- |
| signal generation | | | | 50/50 | HBV/HCV | extracted | 772 | 6 | 39 | - | 60 | 0,62 | var. / m- |
| => | | NFI ratio = 1,0 | | 50/50 | HBV/HCV | extracted | 704 | 7 | 36 | - | 60 | 0,65 | var. / m- |
| else: | 85 : 15 | 5,67 | | 50/50 | HBV/HCV | extracted | 700 | 8 | 36 | - | 60 | 0,62 | var. / m- |
| | 80 : 20 | 4,00 | | 50/50 | HBV/HCV | extracted | 539 | 9 | 38 | - | 60 | 0,57 | var. / m- |
| | 75 : 25 | 3,00 | | 50/50 | HBV/HCV | extracted | 537 | 10 | 37 | - | 60 | 0,55 | var. / m- |
| | 70 : 30 | 2,33 | | 50/50 | HBV/HCV | extracted | 737 | 11 | 37 | - | 60 | 0,61 | var. / m- |
| | 65 : 35 | 1,86 | | 50/50 | HBV/HCV | extracted | 625 | 12 | 33 | - | 60 | 0,64 | var. / m- |
| | 60 : 40 | 1,50 | | 50/5000 | HBV/HCV | extracted | 363 | 13 | 34 | - | 60 | 0,43 | var. / m+ |
| | 55 : 45 | 1,22 | | 50/5000 | HBV/HCV | extracted | 717 | 14 | 32 | - | 60 | 0,50 | var. / m+ |
| | 53 : 47 | 1,13 | | 50/5000 | HBV/HCV | extracted | 639 | 15 | 31 | - | 60 | 0,50 | var. / m+ |
| | | | | 50/5000 | HBV/HCV | extracted | 711 | 16 | 32 | - | 60 | 0,50 | var. / m+ |
| | 47 : 53 | 0,89 | | 50/5000 | HBV/HCV | extracted | 424 | 17 | 34 | - | 60 | 0,37 | var. / m+ |
| | 45 : 55 | 0,82 | | 50/5000 | HBV/HCV | extracted | 492 | 18 | 34 | - | 60 | 0,39 | var. / m+ |
| | 40 : 60 | 0,67 | | 50/5000 | HBV/HCV | extracted | 588 | 19 | 26 | - | 60 | 0,49 | var. / m+ |
| | 35 : 65 | 0,54 | | 50/5000 | HBV/HCV | extracted | 495 | 20 | 32 | - | 60 | 0,41 | var. / m+ |
| | 30 : 70 | 0,43 | | 50/5000 | HBV/HCV | extracted | 425 | 21 | 34 | - | 60 | 0,39 | var. / m+ |
| | 25 : 75 | 0,33 | | 50/5000 | HBV/HCV | extracted | 187 | 22 | 33 | - | 60 | 0,46 | var. / m+ |
| | 20 : 80 | 0,25 | | 50/5000 | HBV/HCV | extracted | 607 | 23 | 26 | - | 60 | 0,51 | var. / m+ |
| | 15 : 85 | 0,18 | | 50/5000 | HBV/HCV | extracted | 667 | 24 | 26 | - | 60 | 0,52 | var. / m+ |

Fig 4b: NFI ratios (Ry / Rx) as f(t)

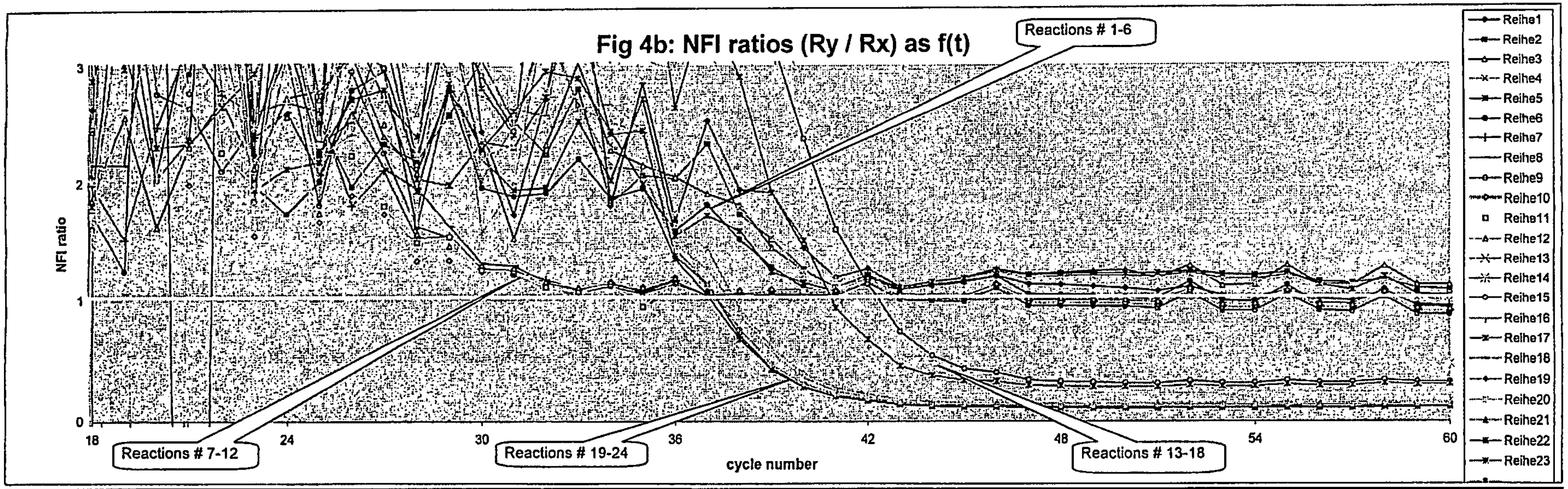


| Ry = JA274 | | Rx = FAM | [cp/ml] | analyte | nucleic acid | SFI (Ry) | valid range (SFI -> AFI) for reaction # = | min | [cycles] | max | NFI ratio (55-60) | / trace |
|---|---|---|---|---|---|---|---|---|---|---|---|---|
| | | | 25 | HBV | extracted | 395 | 1 | 47 | - | 60 | 1,01 | constant |
| Normalization factor = | | 12,00 | 25 | HBV | extracted | 423 | 2 | 46 | - | 60 | 1,15 | borderline |
| => | | | 25 | HBV | extracted | 582 | 3 | 46 | - | 60 | 1,19 | borderline |
| Ry/NF: Rx = 50 : 50 distribution of NFI | | | 25 | HBV | extracted | 448 | 4 | 46 | - | 60 | 1,10 | constant |
| to be expected in case of coupled | | | 25 | HBV | extracted | 495 | 5 | 46 | - | 60 | 1,12 | constant |
| signal generation | | | 25 | HBV | extracted | 605 | 6 | 46 | - | 60 | 0,99 | constant |
| => | | NFI ratio = 1,0 | 10000 | HBV | extracted | 882 | 7 | 39 | - | 60 | 1,00 | constant |
| else: | 85 : 15 | 5,67 | 10000 | HBV | extracted | 843 | 8 | 39 | - | 60 | 0,97 | constant |
| | 80 : 20 | 4,00 | 10000 | HBV | extracted | 772 | 9 | 39 | - | 60 | 0,94 | constant |
| | 75 : 25 | 3,00 | 10000 | HBV | extracted | 743 | 10 | 38 | - | 60 | 0,97 | constant |
| | 70 : 30 | 2,33 | 10000 | HBV | extracted | 971 | 11 | 38 | - | 60 | 0,98 | constant |
| | 65 : 35 | 1,86 | 10000 | HBV | extracted | 868 | 12 | 39 | - | 60 | 0,99 | constant |
| | 60 : 40 | 1,50 | 1:1000/50 | HIV-1-O/HCV | extracted | 74 | 13 | 47 | - | 60 | 0,43 | offset |
| | 55 : 45 | 1,22 | 1:1000/50 | HIV-1-O/HCV | extracted | 84 | 14 | 47 | - | 60 | 0,47 | offset |
| | 53 : 47 | 1,13 | 1:1000/50 | HIV-1-O/HCV | extracted | 98 | 15 | 48 | - | 60 | 0,33 | offset |
| | | | 1:1000/50 | HIV-1-O/HCV | extracted | 84 | 16 | 48 | - | 60 | 0,23 | var. / m- |
| | 47 : 53 | 0,89 | 1:1000/50 | HIV-1-O/HCV | extracted | 75 | 17 | 47 | - | 60 | 0,30 | offset |
| | 45 : 55 | 0,82 | 1:1000/50 | HIV-1-O/HCV | extracted | 81 | 18 | --- | - | --- | 4,25 | #WERT! |
| | 40 : 60 | 0,67 | 1:1000/500 | HIV-1-O/HCV | extracted | 66 | 19 | 46 | - | 60 | 0,14 | var. / m+ |
| | 35 : 65 | 0,54 | 1:1000/500 | HIV-1-O/HCV | extracted | 86 | 20 | 47 | - | 60 | 0,17 | offset |
| | 30 : 70 | 0,43 | 1:1000/500 | HIV-1-O/HCV | extracted | 85 | 21 | 45 | - | 60 | 0,16 | offset |
| | 25 : 75 | 0,33 | 1:1000/500 | HIV-1-O/HCV | extracted | 68 | 22 | 45 | - | 60 | 0,11 | offset |
| | 20 : 80 | 0,25 | 1:1000/500 | HIV-1-O/HCV | extracted | 59 | 23 | 45 | - | 60 | 0,13 | offset |
| | 15 : 85 | 0,18 | 1:1000/500 | HIV-1-O/HCV | extracted | 76 | 24 | 45 | - | 60 | 0,14 | var. / m+ |

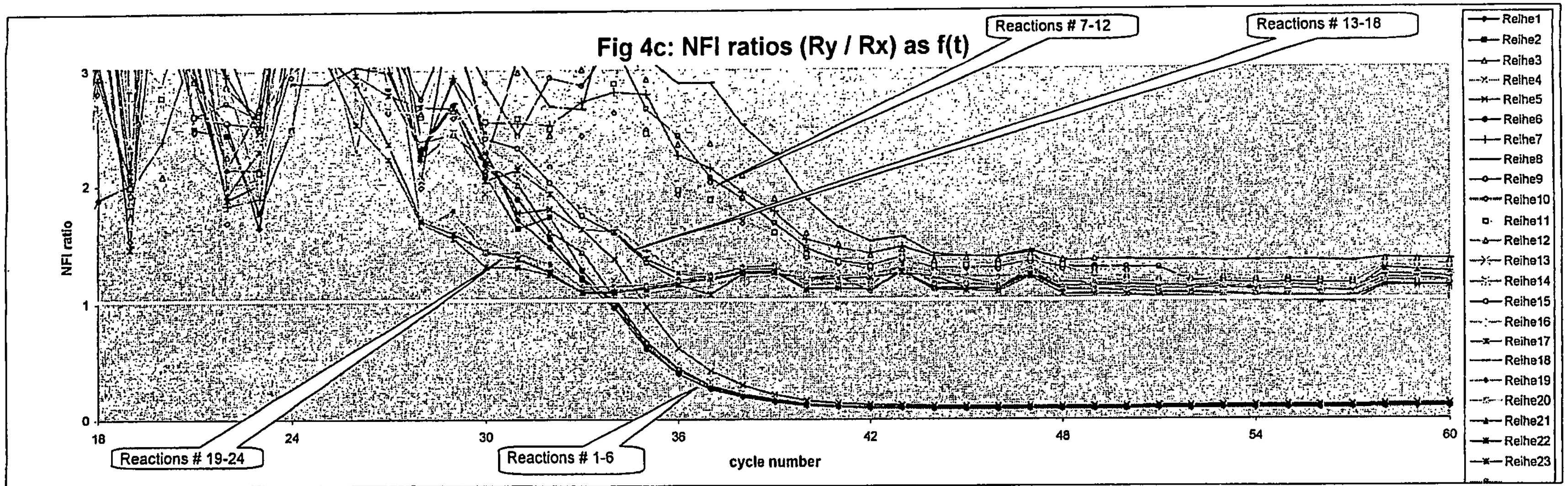


Fig 4c: NFI ratios (Ry / Rx) as f(t)

| Ry = | JA274 | Rx = FAM | [cp/ml] | analyte nucleic acid | | SFI (Ry) | valid range (SFI -> AFI) for reaction # = | min | [cycles] | max | NFI ratio (55-60) / trace | |
|---|---|---|---|---|---|---|---|---|---|---|---|---|
| | | | 1:1000/5000 | HIV-1-O/HCV | extracted | *75* | 1 | 43 | - | 60 | 0,10 | var. / m+ |
| Normalization factor = | | 12,00 | 1:1000/5000 | HIV-1-O/HCV | extracted | *89* | 2 | 43 | - | 60 | 0,11 | var. / m+ |
| => | | | 1:1000/5000 | HIV-1-O/HCV | extracted | *106* | 3 | 43 | - | 60 | 0,11 | var. / m+ |
| Ry/NF: Rx = 50 : 50 distribution of NFI | | | 1:1000/5000 | HIV-1-O/HCV | extracted | *88* | 4 | 43 | - | 60 | 0,12 | var. / m+ |
| to be expected in case of coupled | | | 1:1000/5000 | HIV-1-O/HCV | extracted | *102* | 5 | 43 | - | 60 | 0,12 | var. / m+ |
| signal generation | | | 1:1000/5000 | HIV-1-O/HCV | extracted | *102* | 6 | 43 | - | 60 | 0,11 | var. / m+ |
| => | | NFI ratio = 1,0 | 50/1:1000 | HBV/HIV-1-O | extracted | 538 | 7 | 50 | - | 60 | 1,18 | offset |
| else: | 85 : 15 | 5,67 | 50/1:1000 | HBV/HIV-1-O | extracted | 492 | 8 | 47 | - | 60 | 1,35 | offset |
| | 80 : 20 | 4,00 | 50/1:1000 | HBV/HIV-1-O | extracted | 646 | 9 | 55 | - | 60 | 1,24 | offset |
| | 75 : 25 | 3,00 | 50/1:1000 | HBV/HIV-1-O | extracted | 602 | 10 | 54 | - | 60 | 1,16 | offset |
| | 70 : 30 | 2,33 | 50/1:1000 | HBV/HIV-1-O | extracted | 687 | 11 | 45 | - | 60 | 1,24 | offset |
| | 65 : 35 | 1,86 | 50/1:1000 | HBV/HIV-1-O | extracted | 657 | 12 | 46 | - | 60 | 1,28 | offset |
| | 60 : 40 | 1,50 | 500/1:1000 | HBV/HIV-1-O | extracted | 691 | 13 | 43 | - | 60 | 1,14 | offset |
| | 55 : 45 | 1,22 | 500/1:1000 | HBV/HIV-1-O | extracted | 814 | 14 | 43 | - | 60 | 1,15 | offset |
| | 53 : 47 | 1,13 | 500/1:1000 | HBV/HIV-1-O | extracted | 812 | 15 | 43 | - | 60 | 1,18 | offset |
| | | | 500/1:1000 | HBV/HIV-1-O | extracted | 860 | 16 | 43 | - | 60 | 1,26 | offset |
| | 47 : 53 | 0,89 | 500/1:1000 | HBV/HIV-1-O | extracted | 744 | 17 | 43 | - | 60 | 1,15 | offset |
| | 45 : 55 | 0,82 | 500/1:1000 | HBV/HIV-1-O | extracted | 741 | 18 | 43 | - | 60 | 1,18 | offset |
| | 40 : 60 | 0,67 | 5000/1:1000 | HBV/HIV-1-O | extracted | 847 | 19 | 38 | - | 60 | 1,14 | offset |
| | 35 : 65 | 0,54 | 5000/1:1000 | HBV/HIV-1-O | extracted | 843 | 20 | 39 | - | 60 | 1,12 | constant |
| | 30 : 70 | 0,43 | 5000/1:1000 | HBV/HIV-1-O | extracted | 745 | 21 | 39 | - | 60 | 1,07 | constant |
| | 25 : 75 | 0,33 | 5000/1:1000 | HBV/HIV-1-O | extracted | 896 | 22 | 39 | - | 60 | 1,09 | constant |
| | 20 : 80 | 0,25 | 5000/1:1000 | HBV/HIV-1-O | extracted | 744 | 23 | 39 | - | 60 | 1,07 | constant |
| | 15 : 85 | 0,18 | 5000/1:1000 | HBV/HIV-1-O | extracted | 859 | 24 | 39 | - | 60 | 1,11 | constant |

Fig 4d: NFI ratios (Ry / Rx) as f(t)

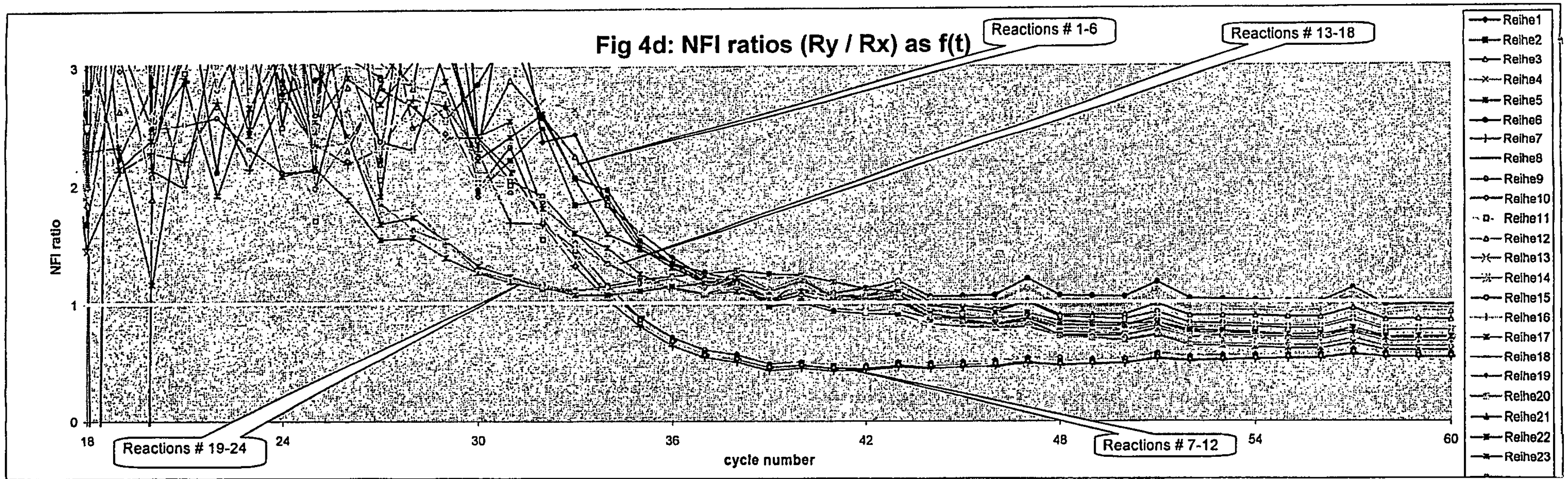


| Ry = | JA274 | Rx = FAM | [cp/ml] | analyte nucleic acid | | SFI (Ry) | valid range (SFI -> AFI) for reaction # | = | min | [cycles] | max | NFI ratio (55-60) / trace | |
|---|---|---|---|---|---|---|---|---|---|---|---|---|---|
| | | | 200/60 | HBV/HCV | extracted | 720 | 1 | | 45 | - | 60 | 0,69 | var. / m- |
| Normalization factor = | | 12,00 | 200/60 | HBV/HCV | extracted | 654 | 2 | | 45 | - | 60 | 0,71 | var. / m- |
| => | | | 200/60 | HBV/HCV | extracted | 704 | 3 | | 44 | - | 60 | 0,88 | offset |
| Ry/NF: Rx = 50 : 50 distribution of NFI | | | 200/60 | HBV/HCV | extracted | 672 | 4 | | 46 | - | 60 | 0,72 | offset |
| to be expected in case of coupled | | | 200/60 | HBV/HCV | extracted | 646 | 5 | | 44 | - | 60 | 0,78 | offset |
| signal generation | | | 200/60 | HBV/HCV | extracted | 670 | 6 | | 44 | - | 60 | 1,01 | constant |
| => | | NFI ratio = 1,0 | 200/2000 | HBV/HCV | extracted | 670 | 7 | | 44 | - | 60 | 0,54 | var. / m+ |
| else: | 85 : 15 | 5,67 | 200/2000 | HBV/HCV | extracted | 706 | 8 | | 44 | - | 60 | 0,53 | var. / m+ |
| | 80 : 20 | 4,00 | 200/2000 | HBV/HCV | extracted | 609 | 9 | | 43 | - | 60 | 0,54 | var. / m+ |
| | 75 : 25 | 3,00 | 200/2000 | HBV/HCV | extracted | 637 | 10 | | 43 | - | 60 | 0,57 | var. / m+ |
| | 70 : 30 | 2,33 | 200/2000 | HBV/HCV | extracted | 787 | 11 | | 44 | - | 60 | 0,56 | var. / m+ |
| | 65 : 35 | 1,86 | 200/2000 | HBV/HCV | extracted | 681 | 12 | | 43 | - | 60 | 0,56 | var. / m+ |
| | 60 : 40 | 1,50 | 1000/60 | HBV/HCV | extracted | 768 | 13 | | 43 | - | 60 | 0,69 | var. / m- |
| | 55 : 45 | 1,22 | 1000/60 | HBV/HCV | extracted | 854 | 14 | | 43 | - | 60 | 0,77 | offset |
| | 53 : 47 | 1,13 | 1000/60 | HBV/HCV | extracted | 862 | 15 | | 42 | - | 60 | 0,80 | offset |
| | | | 1000/60 | HBV/HCV | extracted | 800 | 16 | | 43 | - | 60 | 0,79 | offset |
| | 47 : 53 | 0,89 | 1000/60 | HBV/HCV | extracted | 703 | 17 | | 44 | - | 60 | 0,72 | var. / m- |
| | 45 : 55 | 0,82 | 1000/60 | HBV/HCV | extracted | 799 | 18 | | 43 | - | 60 | 0,71 | var. / m- |
| | 40 : 60 | 0,67 | 10000/60 | HBV/HCV | extracted | 646 | 19 | | 38 | - | 60 | 0,98 | constant |
| | 35 : 65 | 0,54 | 10000/60 | HBV/HCV | extracted | 758 | 20 | | 38 | - | 60 | 0,61 | var. / m- |
| | 30 : 70 | 0,43 | 10000/60 | HBV/HCV | extracted | 735 | 21 | | 39 | - | 60 | 0,95 | constant |
| | 25 : 75 | 0,33 | 10000/60 | HBV/HCV | extracted | 716 | 22 | | 38 | - | 60 | 0,61 | var. / m- |
| | 20 : 80 | 0,25 | 10000/60 | HBV/HCV | extracted | 535 | 23 | | 37 | - | 60 | 0,72 | var. / m- |
| | 15 : 85 | 0,18 | 10000/60 | HBV/HCV | extracted | 771 | 24 | | 38 | - | 60 | 0,64 | var. / m- |

**Fig. 5:** *we consider a panel of potentially ambiguous samples*

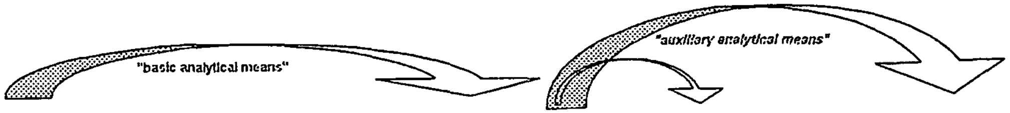


| assignments: | | | AmpDet | |
|---|---|---|---|---|
| P1 | HIV-1-M | R1 / R2; | efficiency: | high |
| P2 | HIV-1-O | R1; | | low |
| P3 | HIV-2 | R2; | | low |
| P4 | HCV | R3; | | high |
| P5 | HBV | R1 / R3; | | high |
| P6 | CMV | R2 / R3; | | medium |
| IC (internal control) | | R4 | | |

*Roughly similar titres are assumed for all infections considered here.*

model data set 3:

| | NFI values | | | | SFI intensities | NFI trace as f(t) | | | Diagnosis |
|---|---|---|---|---|---|---|---|---|---|
| | Ch1 | Ch2 | Ch3 | total | (or differences) | R1/R2 | R1/R3 | R2/R3 | |
| S1 =? | 31 | 30 | 38 | 99 | high | signal in all target-related channels<br>constant | variable / bias <> 1 / early | variable / bias <> 1 / early | *Co-infection HIV-1-M and HCV* |
| | 34 | 33 | 33 | 100 | | | | | |
| | 32 | 35 | 33 | 100 | | | | | |
| | 33 | 32 | 35 | 100 | | | | | |
| | 32 | 30 | 38 | 100 | | | | | |
| | 30 | 33 | 37 | 100 | | | | | |
| S2 = ? | 3 | 15 | 82 | 100 | low-medium | | | variable / late / bias > 1 | *Co-infection HIV-2 and HCV* |
| | 2 | 20 | 78 | 100 | | | | | |
| | 1 | 24 | 75 | 100 | | | | | |
| | 0 | 27 | 73 | 100 | | | | | |
| | 1 | 20 | 79 | 100 | | | | | |
| | 3 | 21 | 76 | 100 | | | | | |
| S3 = ? | 24 | 49 | 27 | 100 | medium | signal in all target-related channels<br>variable / bias < 1 / late | variable / bias > 1 / late | variable / bias > 1 / late | *Co-infection HIV-1-M and HAV* |
| | 21 | 49 | 30 | 100 | | | | | |
| | 28 | 46 | 26 | 100 | | | | | |
| | 27 | 44 | 29 | 100 | | | | | |
| | 25 | 48 | 27 | 100 | | | | | |
| | 22 | 53 | 25 | 100 | | | | | |
| S4 = ? | 52 | 48 | 0 | 100 | high | | variable / late / bias > 1 | | *Co-infection HBV and HIV-1-O* |
| | 50 | 49 | 1 | 100 | | | | | |
| | 47 | 52 | 1 | 100 | | | | | |
| | 50 | 48 | 2 | 100 | | | | | |
| | 49 | 50 | 1 | 100 | | | | | |
| | 50 | 50 | 0 | 100 | | | | | |
| S5 = ? | 46 | 1 | 52 | 99 | low | | variable / late / bias > 1 | | *Co-infection HIV-1-O and HCV* |
| | 48 | 2 | 50 | 100 | | | | | |
| | 45 | 0 | 55 | 100 | | | | | |
| | 49 | 0 | 51 | 100 | | | | | |
| | 46 | 1 | 53 | 100 | | | | | |
| | 47 | 2 | 51 | 100 | | | | | |
| S6 = ? | 40 | 0 | 60 | 100 | high | | variable / early / bias < 1 | | *Co-infection HBV and HCV* |
| | 41 | 1 | 58 | 100 | | | | | |
| | 43 | 1 | 56 | 100 | | | | | |
| | 37 | 2 | 61 | 100 | | | | | |
| | 42 | 3 | 55 | 100 | | | | | |
| | 38 | 0 | 62 | 100 | | | | | |

*legend: - variable / constant pertains to the NFI ratio along the reaction coordinate*
*- early / late denotes the approximation of the final value in case of var. ratios*

NFI distribution; NFI ratios

| | Ch1 | Ch2 | Ch3 |
|---|---|---|---|
| S1 | 32,00 | 32,17 | 35,67 |
| S2 | 1,67 | 21,17 | 77,17 |
| S3 | 24,50 | 48,17 | 27,33 |
| S4 | 49,67 | 49,50 | 0,83 |
| S5 | 46,83 | 1,00 | 52,00 |
| S6 | 40,17 | 1,17 | 58,67 |

*mean values (arithmetic)*
according to reference; deviating

| | Ch1 | Ch2 | Ch3 |
|---|---|---|---|
| S1 | 4,24 | 5,82 | 7,01 |
| S2 | 3,63 | 12,21 | 9,57 |
| S3 | 8,22 | 9,18 | 5,59 |
| S4 | 4,90 | 4,55 | 2,26 |
| S5 | 4,42 | 2,68 | 5,37 |
| S6 | 6,95 | 3,51 | 8,41 |

*threefold standad deviation*

| | Ch1 | Ch2 | Ch3 |
|---|---|---|---|
| P1 | 50 | 50 | 0 |
| P2 | 100 | 0 | 0 |
| P3 | 0 | 100 | 0 |
| P4 | 0 | 0 | 100 |
| P5 | 50 | 0 | 50 |
| P6 | 0 | 50 | 50 |

*NFI reference pattern*

| | Ch1 | Ch2 | Ch3 |
|---|---|---|---|
| P1 | 10,00 | 10,00 | 5,00 |
| P2 | 10,00 | 5,00 | 5,00 |
| P3 | 5,00 | 10,00 | 5,00 |
| P4 | 5,00 | 5,00 | 10,00 |
| P5 | 10,00 | 5,00 | 10,00 |
| P6 | 5,00 | 10,00 | 10,00 |

*10% / 20%-off-set bands pertinent to 100% & 50%-mean values, and factor 5 pertinent to 0% values i.e. accepted ranges for reference pattern*